# EUROPÄISCHE PATENTSCHRIFT

(11) **EP 2 872 480 B1**
(45) Veröffentlichungstag und Bekanntmachung des Hinweises auf die Patenterteilung: **26.09.2018**
(21) Anmeldenummer: 13707664.2
(22) Anmeldetag: 07.03.2013
(51) Int. Cl.: C07C 235/08, C07C 235/10

(54) **LIPASE STABILER VERDICKER**
LIPASE-STABLE THICKENING AGENT
ÉPAISSISSANT STABLE AUX LIPASES

(30) Priorität: 11.07.2012 DE 102012212085
(43) Veröffentlichungstag der Anmeldung: 20.05.2015
(73) Patentinhaber: Evonik Degussa GmbH, 45128 Essen (DE)
(72) Erfinder: KLOSTERMANN, Michael, 45239 Essen (DE); WENK, Hans Henning, 45470 Mülheim an der Ruhr (DE); KÖHLE, Hans-Jürgen, 63533 Mainhausen (DE); VENZMER, Joachim, 45239 Essen (DE); KUPPERT, Dirk, 63739 Aschaffenburg (DE); LATTICH, Jürgen, 61130 Nidderau-Ostheim (DE); KOTTKE, Ulrike, 63589 Linsengericht-Großenhausen (DE)
(86) Internationale Anmeldenummer: PCT/EP2013/054568
(87) Internationale Veröffentlichungsnummer: WO 2014/009027

(56) Entgegenhaltungen:
- WO-A1-95/31961
- WO-A1-98/13017
- WO-A2-2011/130362
- JP-A- JP5- 287 258
- JP-A- 2004 026 781
- US-A- 2 773 852
- US-A- 3 264 281
- US-A1- 2006 047 046
- US-A1- 2006 211 831
- US-A1- 2011 251 294
- NELSON J S ET AL: "CASTOR-BASED DERIVATIVES: SYNTHESIS OF SOME ACRYLATE ESTERS", JOURNAL OF THE AMERICAN OIL CHEMISTS' SOCIETY, SPRINGER, DE, 1. Januar 1965 (1965-01-01), Seiten 542-545, XP000195938, ISSN: 0003-021X

## Beschreibung

### Gebiet der Erfindung

Gegenstand der Erfindung sind neue Verbindungen, wässrige, insbesondere tensidhaltige, Formulierungen enthaltend die neuen Verbindungen sowie die Verwendung der neuen Verbindungen als Verdicker von wässrigen, insbesondere tensidhaltigen Formulierungen.

### Stand der Technik

Einen klassischen Verdicker für tensidische Systeme wie beispielsweise Flüssigwaschmittel stellt hydriertes Rizinusöl dar.
Aus der EP0596209 sind vernetzte Rizinusöl-Derivate bekannt, die ebenfalls Anwendung als Verdicker finden.
In heutigen Waschmitteln werden oft Lipasen, Esterasen und/oder Proteasen als Reinigungsverstärker eingesetzt. Wie in EP2365050 beschrieben ist die Verwendung des hydrierten Rizinusöls in Kombination mit Lipasen jedoch nicht möglich, da dieses durch das Enzym gespalten wird und somit die verdickende Eigenschaft verloren geht.

Die US2773852 beschreibt N-(2-Hydroxypropyl)-12-Hydroxystearamid als antistatisches Additiv in Kunststoffen.
Über die Verwendung von N-(2-Hydroxyethyl)-12-Hydroxystearamid in unterschiedlichen Anwendungen wurde schon in einer Reihe von Patentschriften berichtet. So wird diese Substanz beispielsweise in WO 2010056270 als Additiv in Polymerkompositen, in US 6211139 als tensidischer Zusatz in wässrigen Polyquatformulierungen oder in DE 2434147 als rheologisches Additiv in nichtwässrigen Formulierungen beschrieben. Nicht beschrieben ist bislang hingegen die Verwendung von N-(2-Hydroxyethyl)-12-Hydroxystearamid als Verdicker für wässrige Formulierungen.

In JP 2004-026781 wird N-(N,N-Dimethyl-3-aminopropyl)-12-Hydroxystearamid und N-(N,N-Diethyl-2-aminoethyl)-12-Hydroxystearamid als Konditionierungsagenz in Haarpflegeapplikationen beschrieben.
In der US 3977894 wird die Verwendung von N-(2-Aminoethyl)-12-Hydroxystearamid als Zusatz für organomodifizierte, tonmineralien-basierende Verdicker für unpolare, nicht wässrige Systeme offenbart.
Die WO 01/46373 offenbart überdies das Produkt der Umsetzung von gehärtetem Rizinusöl mit Ethylendiamin und seine Verwendung als Ölphasenverdicker.
In US 2986517 wird über die Verwendung von N,N-di-(2-Hydroxyethyl)-12-Hydroxystearamid als rheologisches Additive in Schmierölen berichtet. US2011/251294 offenbart niedermolekulare Verdickungsmittel. Aufgabe der Erfindung war es, einen Verdicker mit scherverdünnenden Eigenschaften bereitzustellen, welcher darüber hinaus lipasestabil ist.

### Beschreibung der Erfindung

Überraschenderweise wurde gefunden, dass die im Folgenden beschriebenen Amide auf Basis von 12-Hydroxystearinsäure hervorragende Verdicker für wässrige, insbesondere tensidhaltige Formulierungen darstellen und darüber hinaus nicht von Lipasen abgebaut werden.
Gegenstand der vorliegenden Erfindung sind daher bestimmte Amide der 12-Hydroxystearinsäure.
Ein weiterer Gegenstand sind wässrige Formulierungen enthaltend bestimmte Amide der 12-Hydroxystearinsäure.
Noch ein Gegenstand der Erfindung ist die Verwendung von bestimmten Amiden der 12-Hydroxystearinsäure als Verdicker von wässrigen Formulierungen.

Ein Vorteil der vorliegenden Erfindung ist es, dass mit ihr gezielt ein scherverdünnendes rheologisches Verhalten des zu verdickenden Mediums eingestellt werden kann.
Ein weiterer Vorteil der vorliegenden Erfindung ist, dass sie sich durch eine vollständige Stabilität gegenüber Lipasen auszeichnen.

Ein besonderer Vorteil von amino-funktionalisierten Amiden der 12-Hydroxystearinsäure ist überdies, dass ihre verdickenden Eigenschaften über eine Einstellung des pH-Werts reguliert werden können.
Noch ein Vorteil der vorliegenden Erfindung ist die hohe Verdickungswirkung.
Alle angegebenen Prozent (%) sind, wenn nicht anders angegeben, Massenprozent.

Die vorliegende Erfindung umfasst somit eine Verbindung der allgemeinen Formel (I) 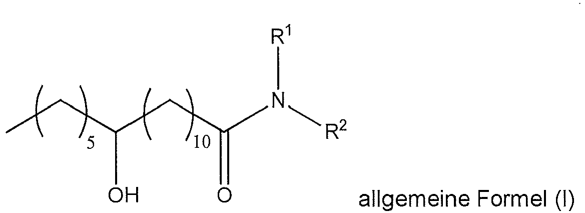 mit
R¹ = N-(2-Hydroxyethyl)-aminoethyl- und R² = N-(2-Hydroxyethyl)-aminoethyl-,
R¹ = 2-(2-Hydroxyethoxy)ethyl- und R² = 2-2-(2-Hydroxyethoxy)ethyl-,
R¹ = Methyl- oder 2,3,4,5,6-pentahydroxyhexyl- und R² = 2,3,4,5,6-pentahydroxyhexylwobei R¹ bevorzugt = Methyl- und
R¹ = H- oder 2-(1-Piperazinyl)ethyl- und R² 2-(1-Piperazinyl)ethyl-, wobei R¹ bevorzugt = H.

Verbindungen der allgemeinen Formel (I) lassen sich beispielsweise darstellen, indem hydriertes Rizinusöl mit einem substituierten Alkylamin unter saurer oder alkalischer Katalyse amidiert wird, ein entsprechender Prozess ist beispielsweise in der DE 19827304 beschrieben. Das hierbei entstehende Glycerin kann im Produkt verbleiben oder kann mindestens teilweise destillativ entfernt werden. Alternativ kann als Fettkomponente auch ein Alkylester des hydrierten Rizinusöls verwendet werden, wobei der bei der Amidierung freiwerdende Alkylalkohol destillativ abgetrennt werden kann.
Ein weiterer möglicher Syntheseweg ist die direkte Amidierung von 12-Hydroxystearinsäure mit substituierten Alkylaminen ggf. unter saurer Katalyse, wobei das entstehende Kondensat destillativ abgetrennt wird; solche Verfahren sind in der EP 2108036 und EP 0574277 beschrieben.

Ein weiterer Gegenstand der vorliegenden Erfindung ist eine wässrige Formulierung enthaltend mindestens eine Verbindung der allgemeinen Formel (I) 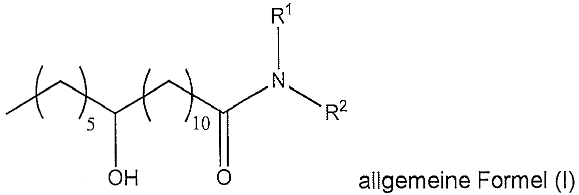 , wobei
unter dem Begriff "wässrige Formulierung" Formulierungen mit einem Wassergehalt von mindestens 20 Gew.-%, insbesondere 40 Gew.-%, besonders bevorzugt 60 Gew.-% bezogen auf die Gesamtformulierung zu verstehen sind,
dadurch gekennzeichnet, dass die Verbindung der allgemeinen Formel (I) ausgewählt ist aus solchen mit
   R¹ = H oder 2-Hydroxypropyl - und R² = 2-Hydroxypropyl-, wobei R¹ bevorzugt = H,
   R¹ = H oder 2-Aminoethyl- und R² = 2-Aminoethyl-, wobei R¹ bevorzugt = H,
   R¹ = H oder N-(2-Hydroxyethyl)-aminoethyl- und R² = N-(2-Hydroxyethyl)-aminoethyl-, wobei R¹ bevorzugt = H,
   R¹ = H oder 2-(2-Hydroxyethoxy)ethyl- und R² = 2-(2-Hydroxyethoxy)ethyl-, wobei R¹ bevorzugt = H,
   R¹ = Methyl- oder 2,3,4,5,6-pentahydroxyhexyl- und R² = 2,3,4,5,6-pentahydroxyhexyl-, wobei R¹ bevorzugt = Methyl- und
   R¹ = H- oder 2-(1-Piperazinyl)ethyl- und R² = 2-(1-Piperazinyl)ethyl-, wobei R¹ bevorzugt = H.

Als besonders vorteilhaft haben sich Formulierungen erwiesen, die dadurch gekennzeichnet sind, dass die Verbindung der allgemeinen Formel (I) ausgewählt ist aus solchen mit
R¹ = H oder 2-Hydroxypropyl - und R² = 2-Hydroxypropyl-, wobei R¹ bevorzugt = H,
R¹ = H oder 2-Aminoethyl- und R² = 2-Aminoethyl-, wobei R¹ bevorzugt = H,

Es ist erfindungsgemäß bevorzugt, dass mindestens eine Verbindung der allgemeinen Formel (I) in einer Menge von 0,1 Gew.-% bis 50 Gew.-%, besonders bevorzugt von 0,5 Gew.-% bis 25 Gew.-%, wobei sich die Gew.-% auf die Gesamtformulierung beziehen, in der wässrigen Formulierung enthalten ist.

Insbesondere bevorzugt sind wässrige, Wasch- und Reinigungsformulierungen, wie z.B. Flüssigwaschmittel oder kosmetische Formulierungen wie Flüssigseifen Duschgele oder Shampoos, welche neben der Verbindung der allgemeinen Formel (I) bevorzugt mindestens ein Tensid enthalten, wobei anionische, nichtionische, kationische und/oder amphotere Tenside eingesetzt werden können. Bevorzugt sind aus anwendungstechnischer Sicht Mischungen aus anionischen und nichtionischen Tensiden. Der Gesamttensidgehalt der wässrigen Formulierung beträgt vorzugsweise 5 bis 60 Gew.-% und besonders bevorzugt 15 bis 40 Gew.-%, bezogen auf die gesamte Formulierung.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C12-C14-Alkohole mit 3 EO, 4 EO oder 7 EO, C9-C11-Alkohol mit 7 EO, C13-C15- Alkohole mit 3 EO, 5 EO, 7 EO oder 8 EO, C12-C18-Alkohole mit 3 EO, 5 EO oder 7 EO und Mischungen aus diesen, wie Mischungen aus C12-C14-Alkohol mit 3 EO und C12-C18-Alkohol mit 7 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf. Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO(Propylenoxid)-Gruppen zusammen im Molekül enthalten, sind einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere.

Selbstverständlich sind auch gemischt alkoxylierte nichtionische Tenside einsetzbar, in denen EO-und PO-Einheiten nicht blockweise, sondern statistisch verteilt sind. Solche Produkte sind durch gleichzeitige Einwirkung von Ethylen- und Propylenoxid auf Fettalkohole erhältlich.

Außerdem können als weitere nichtionische Tenside auch Alkylglykoside eingesetzt werden.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsaurealkylester, vorzugsweise mit 1 bis 4 Kohlenstoffatomen in der Alkylkette, insbesondere Fettsäuremethylester, wie sie beispielsweise in der japanischen Patentanmeldung JP 58/217598 beschrieben sind oder die vorzugsweise nach dem in der internationalen Patentanmeldung WO-A-90/13533 beschriebenen Verfahren hergestellt werden.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide; bei den Polyhydroxyfettsäureamiden handelt es sich um Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Als anionische Tenside werden beispielsweise solche vom Typ der Sulfonate und Sulfate eingesetzt. Als Tenside vom Sulfonat-Typ kommen dabei vorzugsweise C9-C13-Alkylbenzolsulfonate, Olefinsulfonate, d.h. Gemische aus Alken- und Hydroxyalkansulfonaten sowie Disulfonaten, wie man sie beispielsweise aus C12-C18-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C12-C18-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Weitere geeignete anionische Tenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C12-C18-Fettalkohole, beispielsweise aus Kokosfettalkohol, Talgfettalkohol, Lauryl-, Myristyl-, Cetyl- oder Stearylalkohol oder der C10-C20-Oxoalkohole und diejenigen Halbester sekundärer Alkohole dieser Kettenlängen bevorzugt. Weiter-hin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C12-C16-Alkylsulfate und C12-C18-Alkylsulfate sowie C14-C18-Alkylsulfate bevorzugt. Auch 2,3-Alkylsulfate, welche beispielsweise gemäß den US-Patentschriften 3,234,258 oder 5,075,041 hergestellt werden und als Handelsprodukte der Shell Oil Company unter dem Namen DAN® erhalten werden können, sind geeignete anionische Tenside.

Auch die Schwefelsäuremonoester der mit 1 bis 6 Mol Ethylenoxid ethoxylierten geradkettigen oder verzweigten C7-C20-Alkohole, wie 2-Methyl-verzweigte C9-C11-Alkohole mit im Durchschnitt 3,5 Mol Ethylenoxid (EO) oder C12-C18-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.-%, eingesetzt.

Weitere geeignete anionische Tenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C8-C18-Fettalkoholreste oder Mischungen aus diesen. Insbesondere bevorzugte Sulfosuccinate enthalten einen Fettalkoholrest, der sich von ethoxylierten Fettalkoholen ableitet. Dabei sind wiederum Sulfosuccinate, deren Fettalkohol-Reste sich von ethoxylierten Fettalkoholen mit enger Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen. Insbesondere bevorzugte anionische Tenside sind Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.
Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Als amhpotere Tenside können erfindungsgemäß solche oberflächenaktiven Verbindungen eingesetzt werden, die im Molekül mindestens eine quartäre Ammoniumgruppe und mindestens eine -COO-- oder -SO3- -Gruppe tragen. Besonders bevorzugte amphotere Tenside sind in diesem Zusammenhang Betain-Tenside wie Alkyl- oder Alkylamidopropylbetaine. Insbesondere sind hier Betaine wie die N-Alkyl-N,N-dimethylammonium-glycinate, z. B. das Kokosalkyldimethylammoniumglycinat, N-Acyl-aminopropyl-N,N-dimethylammoniumglycinate, z. B. das Kokosacylaminopropyldimethylammoniumglycinat, das C12-C18-Alkyl-dimethyl-acetobetain, das Kokosamidopropyl-dimethyl-acetobetain, 2-Alkyl-3-carboxymethyl-3-hydroxyethyl-imidazoline und Sulfobetaine mit jeweils 8 bis 18 C-Atomen in der Alkyl- oder Acylgruppe sowie das Kokosacylaminoethylhydroxyethylcarboxymethylglycinat bevorzugt. Ein besonders bevorzugtes zwitterionisches Tensid ist das unter der INCI-Bezeichnung Cocamidopropyl Betaine bekannte N,N-Dimethyl-N-(lauroylamidopropyl)ammoniumacetobetain.
Weitere geeignete amphotere Tenside bildet die Gruppe der Amphoacetate und Amphodiacetate, insbesondere beispielsweise Kokos- oder Laurylamphoacetate oderdiacetate, die Gruppe der Amphopropionate und Amphodipropionate sowie die Gruppe der aminosäurebasierten Tenside wie Acylglutamate, insbesondere Disodium Cocoyl Glutamate und Sodium Cocoyl Glutamate, Acylglycinate, insbesondere Cocoyl Glycinate, und Acylsarcosinate, insbesondere Ammonium Lauroyl Sarcosinate und Sodium Cocoyl Sarcosinate.

In einer alternativen Ausführungsform der erfindungsgemäßen Formulierung handelt es sich bei dem Tensid um Biotenside, insbesondere um Glycolipide, wobei Rhamnolipide, Sophorolipide, Trehalose- und andere mycolsäurehaltige Glycolipide sowie Cellobiose- und Mannosylerythritollipide bevorzugt, Sophorolipide und Rhamnolipide besonders bevorzugt und Sophorolipide ganz besonders bevorzugt sind. In diesem Zusammenhang kann es insbesondere vorteilhaft sein, wenn die Formulierung zusätzlich ein Tensid der Gruppe der lineare Alkylbenzolsulfonate (LAS) enthält, insbesondere ausgewählt aus Benzolsulfonsäure,
Dodecylbenzolsulfonsäure, Benzolsulfonsäure C10-13-Alkylderivate, n-Alkyl(C10-C13)Benzolsulfonate, Benzolsulfonsäure-dodecylester, sowie die Natriumsalze der vorgenannten Säuren, besonders bevorzugt Natrium n-C10-13-Alkyl Benzolsulfonat.

Zusätzlich zu der mindestens einen Verbindung der allgemeinen Formel (I) und zu dem mindestens einen Tensid können die wässrigen Formulierungen weitere Inhaltsstoffe enthalten, die die anwendungstechnischen und/oder ästhetischen Eigenschaften der wässrigen Formulierungen weiter verbessern. In der Regel enthalten bevorzugte Formulierungen zusätzlich zu der mindestens einen Verbindung der allgemeinen Formel (I) und zu dem mindestens einen Tensid einen oder mehrere Stoffe ausgewählt aus den Gruppen der Gerüststoffe, Bleichmittel, Bleichaktivatoren, Enzyme, Elektrolyte, nichtwässrigen Lösungsmittel, pH-Stellmittel, Duftstoffe, insbesondere verkapselte Duftstoffe, Parfümträger, Fluoreszenzmittel, Farbstoffe, Hydrotope, Schauminhibitoren, Silikonöle, modifizierte Siloxane, wie organomodifizierte Siloxane, amino- und polyetherfunktionelle und kationische Siloxane, Antiredepositionsmittel, optischen Aufheller, Vergrauungsinhibitoren, Einlaufverhinderer, Knitterschutzmittel, Farbübertragungsinhibitoren, antimikrobiellen Wirkstoffe, Germizide, Fungizide, Antioxidantien, Korrosionsinhibitoren, Antistatika, Bügelhilfsmittel, Phobier- und Imprägniermittel, Depositionspolymere, kationische, aminofunktionelle oder zwitterionische Polymere, Quell- und Schiebefestmittel sowie UV-Absorber.

Erfindungsgemäße kosmetische Pflege- und Reinigungsformulierungen, können zum Beispiel mindestens eine zusätzliche Komponente enthalten, ausgewählt aus der Gruppe der
Emollients,
Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.
Substanzen, die als beispielhafte Vertreter der einzelnen Gruppen eingesetzt werden können, sind dem Fachmann bekannt und können beispielsweise der EP2273966A1 entnommen werden. Diese Patentanmeldung wird hiermit als Referenz eingeführt und gilt somit als Teil der Offenbarung.
Bezüglich weiterer fakultativer Komponenten sowie die eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.
Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Da die vorliegende Erfindung lipasestabile Verdicker behandelt, ist es insbesondere bevorzugt, dass die erfindungsgemäße Formulierung zusätzlich zu der mindestens einen Verbindung der allgemeinen Formel (I) mindestens ein Enzym enthält.
Als Enzyme kommen insbesondere solche aus der Klassen der Hydrolasen, bevorzugt Proteasen, Esterasen, Lipasen, lipolytisch wirkende Enzyme, Amylasen, Cellulasen und andere Glykosylhydrolasen und Gemische der genannten Enzyme in Frage.
Alle diese Hydrolasen tragen in der Wäsche zur Entfernung von Verfleckungen wie
protein-, fett- oder stärkehaltigen Verfleckungen und Vergrauungen bei. Cellulasen und andere Glykosylhydrolasen können darüber hinaus durch das Entfernen von Pilling und Mikrofibrillen zur Farberhaltung und zur Erhöhung der Weichheit des Textils beitragen. Zur Bleiche bzw. zur Hemmung der Farbübertragung können auch Oxireduktasen eingesetzt werden. Besonders gut geeignet sind aus Bakterienstämmen oder Pilzen wie *Bacillus subtilis, Bacillus licheniformis, Streptomyceus griseus* und *Humicola insolens* gewonnene enzymatische Wirkstoffe. Vorzugsweise werden Proteasen vom Subtilisin-Typ und insbesondere Proteasen, die aus *Bacillus lentus* gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease und/oder lipasehaltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder Oxidasen haben sich in einigen Fällen als geeignet erwiesen. Zu den geeigneten Amylasen zählen insbesondere alpha-Amylasen, Iso-Amylasen, Pullulanasen und Pektinasen. Als Cellulasen werden vorzugsweise Cel-Iobiohydrolasen, Endoglucanasen und β-Glucosidasen, die auch Cellobiasen genannt werden, bzw. Mischungen aus diesen eingesetzt. Da sich verschiedene Cellulase-Typen durch ihre CMCase und Avicelase-Aktivitäten unterscheiden, können durch gezielte Mischungen der Cellulasen die gewünschten Aktivitäten eingestellt werden.
Die Enzyme können an Trägerstoffe adsorbiert sein, um sie gegen vorzeitige Zersetzung zu schützen. Der Anteil der Enzyme, Enzymmischungen oder Enzymgranulate kann beispielsweise etwa 0,1 bis 5 Gew.-%, vorzugsweise 0,12 bis etwa 2,5 Gew.-% betragen.

Die erfindungsgemäßen Formulierungen lassen sich nach bekannten Verfahren zubereiten, insbesondere und bevorzugt lassen sie sich durch das im Folgenden beschriebene, erfindungsgemäße Verfahren herstellen:
Ein weiterer Gegenstand der vorliegenden Erfindung ist ein Verfahren zur Herstellung von verdickten, wässrigen, insbesondere tensidhaltigen, Formulierungen umfassend die Verfahrensschritte:
A) Bereitstellen einer Verbindung der allgemeinen Formel (I) 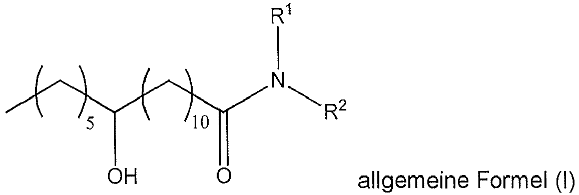
   mit R¹ ausgewählt aus H oder einem gegebenenfalls OH- oder Aminogruppen substituiertem und/oder mit Sauerstoff oder Aminogruppen unterbrochenem Alkylrest mit 1 bis 6 Kohlenstoffatomen und
   mit R² ausgewählt aus H oder einem gegebenenfalls OH- oder Aminogruppen substituiertem und/oder mit Sauerstoff oder Aminogruppen unterbrochenem Alkylrest mit 1 bis 6 Kohlenstoffatomen,
B) Bereitstellen einer wässrigen Formulierung und
C) Mischen der Verbindung der allgemeinen Formel (I) mit der wässrigen Formulierung [X1].

Mit dem erfindungsgemäßen Verfahren lassen sich verdickte Formulierungen herstellen, wobei unter dem Begriff "verdickt" im Zusammenhang mit dem erfindungsgemäßen Verfahren zu verstehen ist, dass die Viskosität, jeweils gemessen bei 25 °C, der wässrigen Formulierung des Verfahrensschrittes C) nach Durchführung des erfindungsgemäßen Verfahrens erhöht ist.

Erfindungsgemäß bevorzugt werden in Verfahrensschritt A) Verbindungen der allgemeine Formel (I) eingesetzt, ausgewählt aus solchen mit
R¹ = H oder 2-Hydroxypropyl - und R² = 2-Hydroxypropyl-, wobei R¹ bevorzugt = H,
R¹ = H oder 2-Hydroxyethyl- und R² = 2-Hydroxyethyl-, wobei R¹ bevorzugt = H,
R¹ = H oder 2-Aminoethyl- und R² = 2-Aminoethyl-, wobei R¹ bevorzugt = H,
R¹ = H oder N-(2-Hydroxyethyl)-aminoethyl- und R² = N-(2-Hydroxyethyl)-aminoethyl-, wobei R¹ bevorzugt = H,
R¹ = H oder 2-(2-Hydroxyethoxy)ethyl- und R² = 2-2-(2-Hydroxyethoxy)ethyl-, wobei R¹ bevorzugt = H,
R¹ = Methyl- oder 2,3,4,5,6-pentahydroxyhexyl- und R² = 2,3,4,5,6-pentahydroxyhexyl-, wobei R¹ bevorzugt = Methyl-,
R¹ = H- oder 2-(1-Piperazinyl)ethyl- und R² = 2-(1-Piperazinyl)ethyl-, wobei R¹ bevorzugt = H und
R¹ = H oder N,N-Dimethyl-3-aminopropyl und R² = N,N-Dimethyl-3-aminopropyl, wobei R¹ bevorzugt = H.

Als besonders vorteilhaft lässt sich in Verfahrensschritt A) solch eine Verbindung der allgemeinen Formel (I) einsetzen, die dadurch gekennzeichnet sind, dass sie ausgewählt ist aus solchen mit
R¹ = H oder 2-Hydroxypropyl - und R² = 2-Hydroxypropyl-, wobei R¹ bevorzugt = H,
R¹ = H oder 2-Hydroxyethyl- und R² = 2-Hydroxyethyl-, wobei R¹ bevorzugt = H,
R¹ = H oder 2-Aminoethyl- und R² = 2-Aminoethyl-, wobei R¹ bevorzugt = H,
R¹ = H oder N,N-Dimethyl-3-aminopropyl und R² = N,N-Dimethyl-3-aminopropyl, wobei R¹ bevorzugt = H.

In Verfahrenesschritt B) werden insbesondere tensidhaltige, wässrige Formulierungen eingesetzt.
Unter dem Begriff "tensidhaltige Formulierung" sind im Zusammenhang mit der vorliegenden Erfindung Formulierungen zu verstehen, die Tenside, insbesondere die oben im Zusammenhang mit den erfindungsgemäßen Formulierungen genannten Tenside, enthalten, vorzugsweise in einer Konzentration von 5 bis 60 Gew.-% und besonders bevorzugt von 15 bis 40 Gew.-%, bezogen auf die gesamte Formulierung. {X1] [dadurch gekennzeichnet, dass in Verfahrensschritt A) eine Verbindung der allgemeinen Formel (I) in Form einer Zusammensetzung aufweisend einen pH-Wert von 0 bis 4, insbesondere von 0,5 bis 2, besonders bevorzugt von 0,8 bis 1,5, eingezetzr wird, wobei mindestens einer der Reste R¹ oder R² ein mit mindestens einer Aminogruppen substituierter Alkylrest mit 1 bis 6 Kohlenstoffatomen ist,
und in einem
Verfahrensschritt D) der pH-Wert der Mischung aus C) auf einen pH-Bereich von 5 bis 12, besonders bevorzugt auf einen pH-Bereich von 7 bis 10, erhöht wird.]

Im Zusammenhang mit der vorliegenden Erfindung ist der pH-Wert bei 25 °C mit einer kalibrierten pH-Elektrode gemäß ISO 4319 (1977) zu messen.

In dieser Ausführungsform wird in Verfahrensschritt A) insbesondere eine Verbindung der allgemeinen Formel (I) eingesetzt, die dadurch gekennzeichnet ist, dass sie ausgewählt ist aus solchen mit
R¹ = H oder N-(2-Hydroxyethyl)-aminoethyl- und R² = N-(2-Hydroxyethyl)-aminoethyl-, wobei R¹ bevorzugt = H,
R¹ = H oder 2-Aminoethyl- und R² = 2-Aminoethyl-, wobei R¹ bevorzugt = H und
R¹ = H oder N,N-Dimethyl-3-aminopropyl und R² = N,N-Dimethyl-3-aminopropyl, wobei R¹ bevorzugt = H.

Die Zusammensetzung enthaltend die Verbindung der allgemeinen Formel (I), die in Verfahrensschritt A) bereitgestellt wird, ist insbesondere eine wässrige Zusammensetzung.

In dieser Ausführungsform wird in Verfahrensschritt D) der pH-Wert durch Zugabe einer Base erhöht. Bevorzugt eingesetzte Basen sind ausgewählt aus NaOH, KOH, Ammoniak, Monoethanolamin, Diethanolamin und Triethanolamin, wobei Monoethanolamin besonders bevorzugt ist.

In einer alternativen bevorzugten Ausführungsform des erfindungsgemäßen Verfahren zur Verdickung von wässrigen Formulierungen, insbesondere zur Verdickung von tensidhaltigen, wässrigen Formulierungen wird in
Verfahrensschritt B) das Mischen in einem Temperaturbereich von 65 °C bis 100 °C, bevorzugt von 75 °C bis 85 °C durchgeführt und in einem
Verfahrensschritt E) die Mischung aus C) auf einen Temperatur-Bereich von 0 °C bis 60 °C, besonders bevorzugt auf einen Temperatur-Bereich von 5 °C bis 45 °C, abgesenkt.

In diesem Zusammenhang wird in Verfahrensschritt A) insbesondere eine Verbindung der allgemeinen Formel (I) eingesetzt, die dadurch gekennzeichnet ist, dass sie ausgewählt ist aus solchen mit
R¹ = H oder 2-Hydroxypropyl - und R² = 2-Hydroxypropyl-, wobei R¹ bevorzugt = H und
R¹ = H oder 2-Hydroxyethyl- und R² = 2-Hydroxyethyl-, wobei R¹ bevorzugt = H,

Ein weiterer Gegenstand der vorliegenden Erfindung ist die Verwendung einer Verbindung der allgemeinen Formel (I) 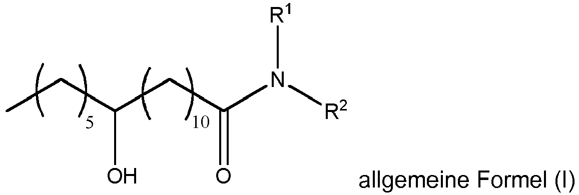
mit R¹ ausgewählt aus H oder einem gegebenenfalls OH- oder Aminogruppen substituiertem und/oder mit Sauerstoff oder Aminogruppen unterbrochenem Alkylrest mit 1 bis 6 Kohlenstoffatomen und
mit R² ausgewählt aus H oder einem gegebenenfalls OH- oder Aminogruppen substituiertem und/oder mit Sauerstoff oder Aminogruppen unterbrochenem Alkylrest mit 1 bis 6 Kohlenstoffatomen, zur Verdickung von wässrigen Formulierungen, insbesondere zur Verdickung von tensidhaltigen, wässrigen Formulierungen.

Erfindungsgemäß bevorzugt werden Verbindungen der allgemeine Formel (I) verwendet, ausgewählt aus solchen mit
R¹ = H oder 2-Hydroxypropyl - und R² = 2-Hydroxypropyl-, wobei R¹ bevorzugt = H,
R¹ = H oder 2-Hydroxyethyl- und R² = 2-Hydroxyethyl-, wobei R¹ bevorzugt = H,
R¹ = H oder 2-Aminoethyl- und R² = 2-Aminoethyl-, wobei R¹ bevorzugt = H,
R¹ = H oder N-(2-Hydroxyethyl)-aminoethyl- und R² = N-(2-Hydroxyethyl)-aminoethyl-, wobei R¹ bevorzugt = H,
R¹ = H oder 2-(2-Hydroxyethoxy)ethyl- und R² = 2-2-(2-Hydroxyethoxy)ethyl-, wobei R¹ bevorzugt = H,
R¹ = Methyl- oder 2,3,4,5,6-pentahydroxyhexyl- und R² = 2,3,4,5,6-pentahydroxyhexyl-, wobei R¹ bevorzugt = Methyl-,
R¹ = H- oder 2-(1-Piperazinyl)ethyl- und R² = 2-(1-Piperazinyl)ethyl-, wobei R¹ bevorzugt = H und
R¹ = H oder N,N-Dimethyl-3-aminopropyl und R² = N,N-Dimethyl-3-aminopropyl, wobei R¹ bevorzugt = H.

Unter dem Begriff "tensidhaltige Formulierung" sind im Zusammenhang mit der vorliegenden Erfindung Formulierungen zu verstehen, die insbesondere die oben im Zusammenhang mit den erfindungsgemäßen Formulierungen genannten Tenside enthalten, vorzugsweise in einer Konzentration von 5 bis 60 Gew.-% und besonders bevorzugt von 15 bis 40 Gew.-%, bezogen auf die gesamte Formulierung.

Als besonders vorteilhaft lässt sich solch eine Verbindung der allgemeinen Formel (I) verwenden, die dadurch gekennzeichnet ist, dass sie ausgewählt ist aus solchen mit
R¹ = H oder 2-Hydroxypropyl - und R² = 2-Hydroxypropyl-, wobei R¹ bevorzugt = H,
R¹ = H oder 2-Hydroxyethyl- und R² = 2-Hydroxyethyl-, wobei R¹ bevorzugt = H,
R¹ = H oder 2-Aminoethyl- und R² = 2-Aminoethyl-, wobei R¹ bevorzugt = H,
R¹ = H oder N,N-Dimethyl-3-aminopropyl und R² = N,N-Dimethyl-3-aminopropyl, wobei R¹ bevorzugt = H.

In einer bevorzugten Ausführungsform der erfindungsgemäßen Verwendung einer Verbindung der allgemeinen Formel (I) zur Verdickung von wässrigen Formulierungen, insbesondere zur Verdickung von tensidhaltigen, wässrigen Formulierungen ist mindestens einer der Reste R¹ oder R² ein mit mindestens einer Aminogruppen substituierter Alkylrest mit 1 bis 6 Kohlenstoffatomen, wobei die erfindungsgemäße Verwendung eine pH-Wert-Änderung der wässrigen Formulierung, insbesondere eine pH-Wert Erhöhung, bevorzugt auf einen pH-Bereich von 5 bis 12, besonders bevorzugt auf einen pH-Bereich von 7 bis 10, umfasst. In diesem Zusammenhang wird insbesondere eine Verbindung der allgemeinen Formel (I) verwendet, die dadurch gekennzeichnet ist, dass sie ausgewählt ist aus solchen mit
R¹ = H oder N-(2-Hydroxyethyl)-aminoethyl- und R² = N-(2-Hydroxyethyl)-aminoethyl-, wobei R¹ bevorzugt = H,
R¹ = H oder 2-Aminoethyl- und R² = 2-Aminoethyl-, wobei R¹ bevorzugt = H und
R¹ = H oder N,N-Dimethyl-3-aminopropyl und R² = N,N-Dimethyl-3-aminopropyl, wobei R¹ bevorzugt = H.

In einer alternativen bevorzugten Ausführungsform der erfindungsgemäßen Verwendung einer Verbindung der allgemeinen Formel (I) zur Verdickung von wässrigen Formulierungen, insbesondere zur Verdickung von tensidhaltigen, wässrigen Formulierungen umfasst die erfindungsgemäße Verwendung eine Temperatur-Senkung der wässrigen Formulierung, bevorzugt auf einen Temperatur-Bereich von 0 bis 60 °C, besonders bevorzugt auf einen Temperatur-Bereich von 5 bis 45 °C. In diesem Zusammenhang wird insbesondere eine Verbindung der allgemeinen Formel (I) verwendet, die dadurch gekennzeichnet ist, dass sie ausgewählt ist aus solchen mit
R¹ = H oder 2-Hydroxypropyl - und R² = 2-Hydroxypropyl-, wobei R¹ bevorzugt = H und
R¹ = H oder 2-Hydroxyethyl- und R² = 2-Hydroxyethyl-, wobei R¹ bevorzugt = H,

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Die in den Bespielen aufgeführten Rheologie-Experiment (Viskosität als Funktion der Schubspannung in einem Schubspannungsbereich von 0,1 bis 50 Pa) wurden mit einem luftgelagertem Oszillationsrheometer (Modell Stresstech der Firma Rheologica) durchgeführt, wobei in allen Fällen mit einer Platte-Platte-Geometrie bei einem Spaltabstand von 1,0 mm gemessen wurde. Alle Messungen wurden bei 25°C ausgeführt.

Folgende Abbildungen sind Bestandteil der Beispiele:
Abbildung 1: Rheologie-Profil einer Waschmittelformulierung enthaltend N-(2-Hydroxyethyl)-12-Hydroxystearamid
Abbildung 2: Rheologie-Profil einer Waschmittelformulierung enthaltend N-(2-Hydroxypropyl)-12-Hydroxystearamid
Abbildung 3: Rheologie-Profil einer Waschmittelformulierung enthaltend N-(2-Hydroxypropyl)-12-Hydroxystearamid (2)
Abbildung 4: Rheologie-Profil einer Waschmittelformulierung enthaltend N-(2-aminoethyl)-12-Hydroxystearamid
Abbildung 5: Rheologie-Profil einer Waschmittelformulierung enthaltend N-(N,N-Dimethyl-3-aminopropyl)-12-Hydroxystearamid
Abbildung 6: Rheologie-Profil einer wässrigen Formulierung enthaltend N-(2,3,4,5,6-pentahydroxyhexyl)-12-Hydroxystearamid
Abbildung 7: Rheologie-Profil einer Waschmittelformulierung enthaltend hydriertes Rizinusöl
Abbildung 8: Lipasestabilität einer Waschmittelformulierung enthaltend N-(2-Hydroxypropyl)-12-Hydroxystearamid im Vergleich zu hydriertem Rizinusöl nach 60 Minuten (8a) und nach sechs Tagen (8b).

### Beispiele:

### Beispiel 1: Synthese von N-(2-Hydroxyethyl)-12-Hydroxystearamid

824 g (0,88 mol) hydriertes Rizinusöl wurden bei 90 °C unter Rühren mit 161 g (2,64 mol) 2-Hydroxyethylamin versetzt und homogenisiert. Diese Mischung wurde bei einem Vakuum von 50 mbar und einer Temperatur von 90 - 100 °C für 30 Minuten getrocknet. Anschließend wurde das Vakuum mit Stickstoff gebrochen und 10 g einer 30 Gew.-%igen Natrium-Methanolat-Lösung in Methanol wurden zugegen. Das Reaktionsgemisch wurde 3 Stunden bei 110 °C gerührt, die Reaktionskontrolle erfolgte über die Bestimmung des freien 2-Hydroxyethylamins (Säure-Base-Titration). Es wurden 989 g einer N-(2-Hydroxyethyl)-12-Hydroxystearamid erhalten, der Gehalt an freiem 2-Hydroxyethylamin betrug 1,2 Gew.-%.

### Beispiel 2: Synthese von N-(2-Hydroxypropyl)-12-Hydroxystearamid

374,2 g (0,40 mol) hydriertes Rizinusöl wurden wie unter Beispiel 1 beschrieben mit 90,1 g (1,2 mol) 2-Hydroxypropylamin gemischt und getrocknet. Zu dieser Reaktionsmischung wurden 5 g einer 30 Gew.-%igen Natrium-Methanolat-Lösung gegeben und es wurde für 2,5 Stunden bei 110 °C gerührt. Die Reaktionskontrolle erfolgte über die Bestimmung des freien 2-Hydroxypropylamin (Säure-Base-Titration). Hierbei reagierte das Reaktionsgemisch bis auf einen 2-Hydroxypropylamin-Gehalt von 0,8 Gew.-% ab.

### Beispiel 3: Synthese von N,N-Di-(2-Hydroxyethyl)-12-Hydroxystearamid

875,3 g (0,94 mol) hydriertes Rizinusöl wurden wie unter Beispiel 1 beschrieben mit 295,1 g (2,81 mol) N,N-Di-(2-hydroxyethyl)amin gemischt und getrocknet. Zu dieser Reaktionsmischung wurden 15 g einer 30 Gew.-%igen Natrium-Methanolat-Lösung gegeben und es wurde für 3 Stunden bei 110 °C gerührt. Die Reaktionskontrolle erfolgte über die Bestimmung des freien N,N-(Di-2-hydroxyethyl)amin. Hierbei reagierte das Reaktionsgemisch bis auf einen N,N-Di-(2-hydroxyethyl)amin -Gehalt von 4,0 Gew.-% ab.

### Beispiel 4: Synthese von N-(2-Aminoethyl) -12-Hydroxystearamid

561 g (0,6 mol) hydriertes Rizinusöl wurden wie unter Beispiel 1 beschrieben mit 541 g (9,0 mol) 2-Aminoethylamin und 14 g einer 30 Gew.-%igen Natrium-Methanolat-Lösung zur Reaktion gebracht. Nach beendeter Amidierung wird der Überschuss Ethylendiamin bei 160 °C/20 mbar abdestilliert. Das resultierende Amid hat ein Restgehalt von 7,4 % 2-Aminoethylamin.

### Beispiel 5: Synthese von N-2-(2-Hydroxyethoxy)ethyl-12-Hydroxystearamid

882,3 g (0,943 mol) hydriertes Rizinusöl wurden wie unter Beispiel 1 beschrieben mit 297,4 g (2,83 mol) N-2-(2-Hydroxyethoxy)ethylamin und 18 g einer 30 Gew.-%igen Natrium-Methanolat-Lösung zur Reaktion gebracht. Hierbei reagierte das Reaktionsgemisch bis auf einen Rest-Amin-Gehalt von 4,6 Gew.-% ab.

### Beispiel 6: Synthese von N-(2,3,4,5,6-pentahydroxyhexyl)-12-Hydroxystearamid

374 g (0,4 mol) hydriertes Rizinusöl wurden wie unter Beispiel 1 beschrieben mit 234,2 g (1,2 mol) N-(2,3,4,5,6-pentahydroxyhexyl)-amin und 9 g einer 30 Gew.-%igen Natrium-Methanolat-Lösung zur Reaktion gebracht. Hierbei reagierte das Reaktionsgemisch bis auf einen Rest-Amin -Gehalt von 5 Gew.-% ab.

### Beispiel 7: Synthese von N-(N,N-Dimethyl-3-aminopropyl)-12-Hydroxystearamid

374,2 g (0,40 mol) hydriertes Rizinusöl wurden bei 90 °C vorgelegt und 30 min bei einem Vakuum von 50 mbar getrocknet. Anschließend wurde das Vakuum mit Stickstoff gebrochen und 0,25 g hypophosphorige Säure (50 Gew.-%ig in Wasser) sowie 159,4 g (1,560 mol) N,N-Dimethyl-3-aminopropylamin wurden zugegeben. Das Reaktionsgemisch wurde für 3 h bei 120 °C gerührt. Die Reaktionskontrolle erfolgte über IR-Spektroskopie, wobei die Reaktion bis zum vollständigen verschwinden der Esterbande umgesetzt wurde. Anschließend wurde die Temperatur auf 140 ° C erhöht und der Überschuss an N,N-Dimethyl-3-aminopropylamin bei einem Vakuum von 20 mbar abdestilliert. Es wurden 496 g N-(N,N-Dimethyl-3-aminopropyl)-12-Hydroxystearamid erhalten.

### Beispiel 8: N-(2-Hydroxyethyl)-12-Hydroxystearamid

Eine wässrige Zusammensetzung von 3 Gew.-% N-(2-Hydroxyethyl)-12-Hydroxystearamid sowie 10 Gew.-% eines mit Monoethanolamin neutralisierten linearen Alkylbenzolsulfonats wurde erstellt und unter Rühren (Magnetrührkern) auf 80 °C erwärmt, bis eine homogene Lösung vorlag. Danach wurde langsam unter Rühren auf Raumtemperatur abgekühlt (Abkühlrate ∼ 2 °C pro Minute).
33 Gew.-% dieser Mischung wurden in eine Waschmittelzusammensetzung gemäß Tabelle 1 eingearbeitet, so dass eine 1 Gew.-%ige Formulierung an N-(2-Hydroxyethyl)-12-Hydroxystearamid erhalten wurde:

**Tabelle 1**

| Inhaltsstoff | Gew.-% |
|---|---|
| Lineares Alkylbenzolsulfonats | 9.0 |
| C12-14 Fettalkoholethoxylat | 4.5 |
| C12-18 Fettsäure | 3.8 |
| C12-14 Dimethylaminoxid | 1.5 |
| Zitronensäure | 1.5 |
| 1,2 Propandiol | 3.8 |
| Borsäure | 0.4 |
| Ethanol | 1.1 |
| Wasser | auf 100% |
| Monoethanolamin | auf pH = 8.0 |

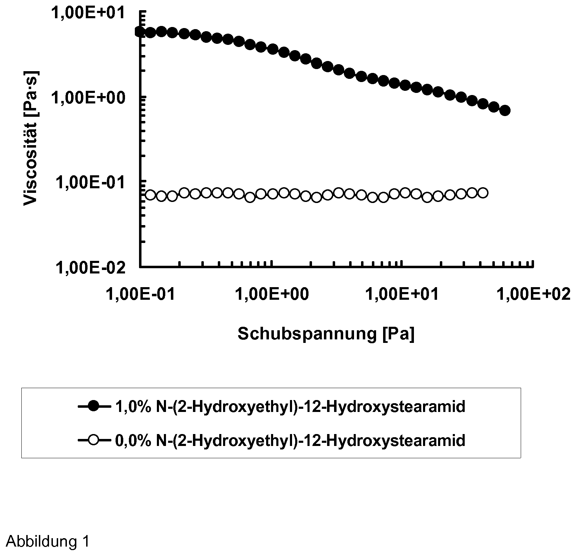

Die Formulierung weist, wie in Abbildung 1 gezeigt, im Rheologie-Test scherverdünnende Eigenschaften auf.

### Beispiel 9: N-(2-Hydroxypropyl)-12-Hydroxystearamid

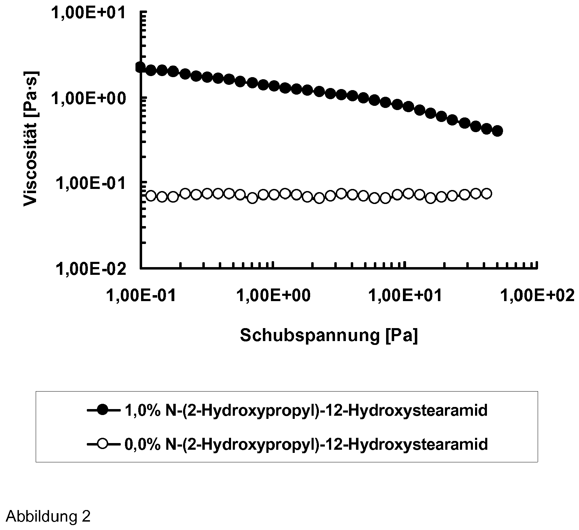

Eine wässrige Zusammensetzung von 4 Gew.-% N-(2-Hydroxypropyl)-12-Hydroxystearamid sowie 19,1 Gew.-% eines mit Monoethanolamin neutralisierten linearen Alkylbenzolsulfonats wurde erstellt und unter Rühren (Magnetrührkern) auf 80 °C erwärmt, bis eine homogene Lösung vorlag. Danach wurde langsam unter Rühren auf Raumtemperatur abgekühlt (Abkühlrate (∼ 2 °C pro Minute). 25 Gew.-% dieser Mischung wurden in eine Waschmittelzusammensetzung gemäß Tabelle 1 eingearbeitet, so dass eine 1 Gew.-%ige Formulierung an N-(2-Hydroxypropyl)-12-Hydroxystearamid erhalten wurde. Die Formulierung weist, wie in Abbildung 2 gezeigt, im Rheologie-Test scherverdünnende Eigenschaften auf.

In einer alternativen Herangehensweise wurde N-(2-Hydroxypropyl)-12-Hydroxystearamid direkt in die Beispielformulierung gemäß Tabelle 1 eingearbeitet, bis eine Endkonzentration von 1,00 Gew.-% eingestellt wurde. Die Formulierung wurde unter Rühren (Magnetrührkern) auf 80 °C erwärmt und langsam unter Rühren auf Raumtemperatur abgekühlt (Abkühlrate ∼ 2 °C pro Minute).

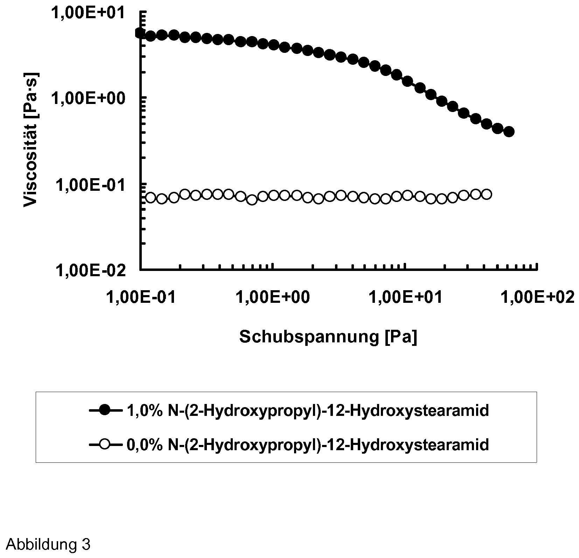

Auch hier weist die Formulierung, wie in Abbildung 3 gezeigt, im Rheologie-Test scherverdünnende Eigenschaften auf.

### Beispiel 10: N-(2-aminoethyl)-12-Hydroxystearamid

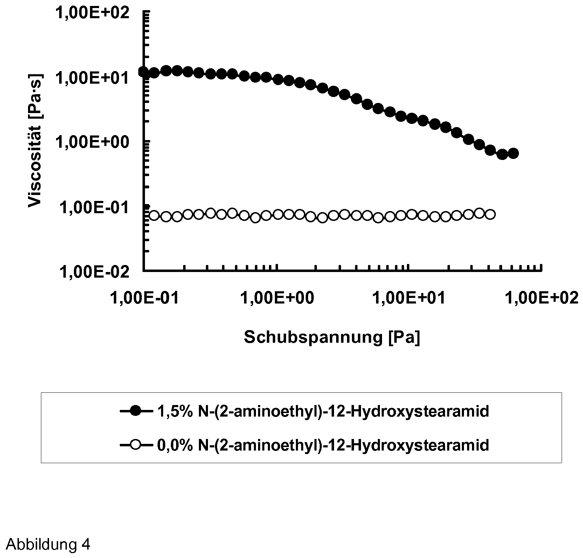

In die in Tabelle 1 beschrieben Waschmittelformulierung wurden 1,5 Gew.-% : N-(2-aminoethyl)-12-Hydroxystearamid eingearbeitet. Die Formulierung wurde unter Rühren (Magnetrührkern) auf 80 °C erwärmt und langsam unter Rühren auf Raumtemperatur abgekühlt (Abkühlrate ∼ 2 °C pro Minute). Wie in Abbildung 4 gezeigt weist die Formulierung im Rheologie-Test scherverdünnende Eigenschaften auf.

### Beispiel 11: N-(N,N-Dimethyl-3-aminopropyl)-12-Hydroxystearamid

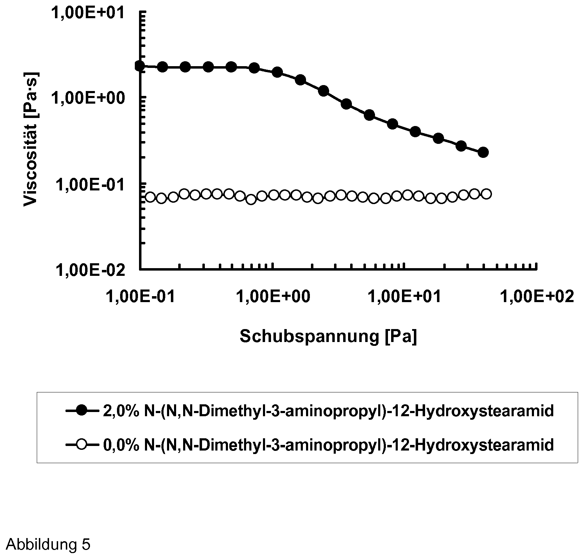

Eine wässrige, salzsaure Lösung (pH = 1) von 3,5 Gew.-% N-(N,N-Dimethyl-3-aminopropyl)-12-Hydroxystearamid wurde bei Raumtemperatur unter langsamem Rühren (Magnetrührkern) hergestellt. In diese Lösung wurde die in Tabelle 1 beschriebene Waschmittelzusammensetzung eingearbeitet, sodass eine Endkonzentration von 2 Gew.-% N-(N,N-Dimethyl-3-aminopropyl)-12-Hydroxystearamid bezogen auf die Gesamtformulierung erhalten wurde. Im Anschluss wurde der pH-Wert der Formulierung mit Monoethanolamine auf pH = 8 eingestellt, wobei ein deutlicher Anstieg der Viskosität der Formulierung beobachtet werden konnte. Wie in Abbildung 5 gezeigt weist die Formulierung im Rheologie-Test scherverdünnende Eigenschaften auf.

### Beispiel 12: N-(2,3,4,5,6-pentahydroxyhexyl)-12-Hydroxystearamid

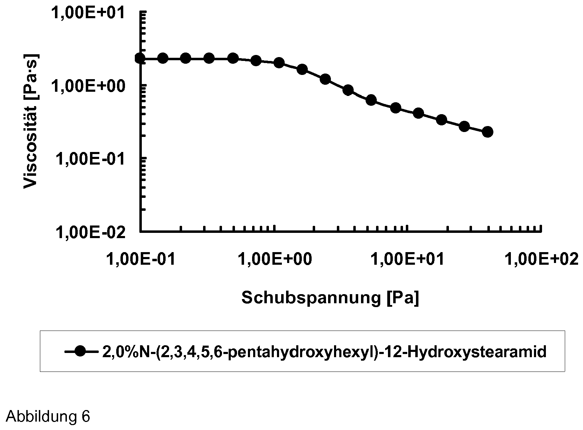

Eine wässrige Zusammensetzung von 2 Gew.-% N-(2,3,4,5,6-pentahydroxyhexyl)-12-Hydroxystearamid wurde erstellt und unter Rühren (Magnetrührkern) auf 80 °C erwärmt, bis eine homogene Lösung vorlag. Danach wurde langsam unter Rühren auf Raumtemperatur abgekühlt (Abkühlrate (∼ 2 °C pro Minute). Wie in Abbildung 6 gezeigt weist die Formulierung im Rheologie-Test scherverdünnende Eigenschaften auf.

### Beispiel 13: Vergleichsbeispiel: hydriertes Rizinusöl

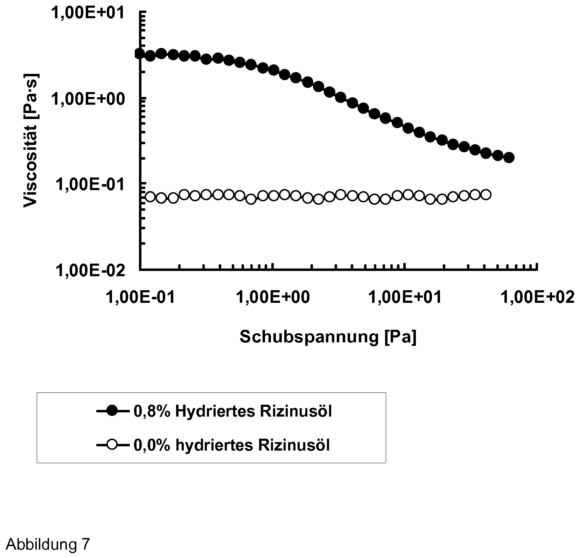

Eine wässrige Zusammensetzung von 4 Gew.-% hydriertem Rizinusöl sowie 19,1 Gew.-% eines mit Monoethanolamin neutralisierten linearen Alkylbenzolsulfonats wurde erstellt und unter Rühren (Magnetrührkern) auf 80 °C erwärmt, bis eine homogene Lösung vorlag. Danach wurde langsam unter Rühren auf Raumtemperatur abgekühlt (Abkühlrate (∼ 2 °C pro Minute). 20 Gew.-% dieser Mischung wurden in eine Waschmittelzusammensetzung gemäß Tabelle 1 eingearbeitet, so dass eine 0,8 Gew.-%ige Formulierung an hydriertem Rizinusöl erhalten wurde. Die Formulierung weist, wie in Abbildung 7 gezeigt, im Rheologie-Test scherverdünnende Eigenschaften auf.

### Beispiel 14: Lipasestabilität von N-(2-Hydroxypropyl)-12-Hydroxystearamid

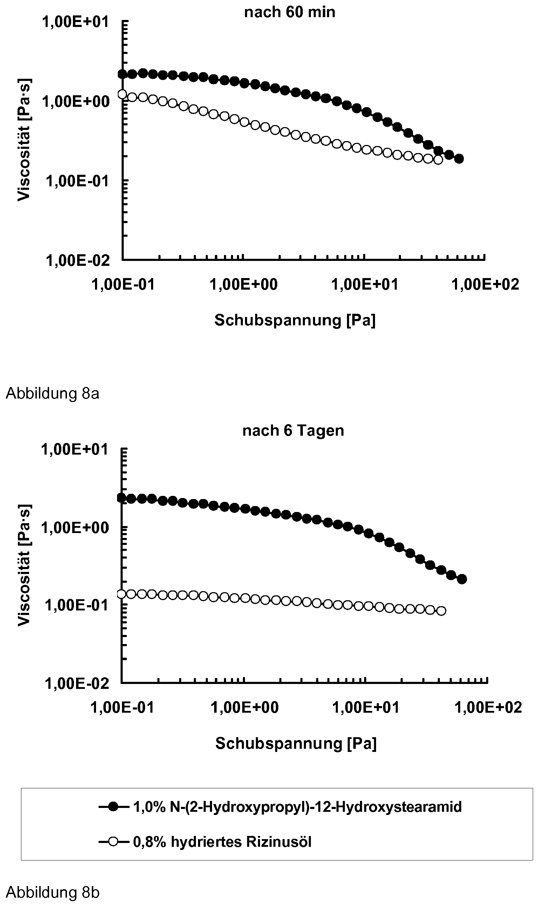

Zur erstgenannten Beispielformulierung des Beispieles 2 wurde 1 Gew.-% Lipase (Lypozyme) zugefügt. Als Vergleichsformulierung diente die im Vergleichsbeispiel beschriebene Formulierung, zu welcher ebenfalls 1 Gew.-% Lipase zugefügt wurde. Das Rheologieverhalten der Formulierungen wurde eine Stunde und sechs Tage nach Anmischung aufgenommen; wie in Abbildung 8a und b gezeigt bricht die verdickende Eigenschaft des hydrierten Rizinusöls nach sechs Tagen komplett zusammen, wohingegen N-(2-Hydroxypropyl)-12-Hydroxystearamid seine Gesamtaktivität beibehält.

### Beispiel 15: Verdickung einer Biotensid-Formulierung

Folgende Formulierungen wurden erstellt und deren Viskositäten mit einem Brookfield Viscosimeter LVT, ausgestattet mit Spindel 2, bei 30 upm und einer Temperatur von 25°C gemessen:
SL = Sophorolipid der Firma Ecover.
LAS = MARLON ARL der Firma Sasol, Natrium-(n-C10-C13-Alkylbenzolsulfonat)

| LAS [Gew.-%] | SL [Gew.-%] | N-(2-Hydroxyethyl)-12-Hydroxystearamid [Gew.-%] | Viscosität [mPas] |
|---|---|---|---|
| 4,5 | 4,5 | 0 | 25 |
| 4,5 | 4,5 | 1 | 1000 |
| 8,9 | 0,1 | 1 | 2500 |
| 8,1 | 0,9 | 1 | 3000 |
| 6,8 | 2,2 | 1 | 7500 |

Die Ergebnisse zeigen, dass die erfindungsgemäße Verbindung in der Lage ist, eine biotensidische Formulierung zu verdicken.

### Beispiel 16: Formulierungsbeispiele

Die in den nachfolgenden Tabellen 2 bis 29 angegebenen Formulierungsbeispiele zeigen exemplarische Vertreter einer Vielzahl von möglichen erfindungsgemäßen Formulierungen.

Falls die Herstellung der Formulierung zuvor die getrennte Zubereitung bzw. Mischung von Formulierungsbestandteilen erfordert, wird dieses als mehrphasige Zubereitung bezeichnet.

Falls eine zweiphasige Herstellung erforderlich ist, werden die beiden Phasen mit A und B in den angegebenen Tabellen gekennzeichnet. Bei dreiphasigen Prozessen werden die drei Phasen mit A, B und C benannt. Wenn nicht anders angegeben handelt es sich bei den Angaben um Angaben in Gew.-%.

In den aufgeführten Beispielen wird das entsprechende 12-Hydroxystearinsäure-Amid stets bei einer Temperatur von 80°C in die Endformulierung eingearbeitet.

**Tabelle 2: Formulierungsbeispiel 1, Shampoo, PEG- & Sulfat frei.**

| | |
|---|---|
| REWOTERIC® AMC, Evonik Goldschmidt GmbH, 32%-ig, (INCI: Sodium Cocoamphoacetate) | 15,00 |
| REWOPOL® SB F 12 P, Evonik Goldschmidt GmbH, 96%-ig, (INCI: Disodium Lauryl Sulfosuccinate) | 3,80 |
| Perfume | 0,30 |
| Water | 65,10 |
| TEGO® Betain F 50, Evonik Goldschmidt GmbH, 38%-ig, (INCI: Cocamidopropyl Betaine) | 13,00 |
| ANTIL® HS 60, Evonik Goldschmidt GmbH, (INCI: Cocamidopropyl Betaine; Glyceryl Laurate) | 1,00 |
| Citric Acid, 30-ig% | q.s. |
| Preservative | 0,30 |
| N-(N,N-Dimethyl-3-aminopropyl)-12-Hydroxystearamid | 1,5 |

**Tabelle 3: Formulierungsbeispiel 2, mildes Haar- und Körperreinigungsmittel**

| | |
|---|---|
| Plantacare® 1200 UP, Cognis, 50%-ig, (INCI: Lauryl Glucoside) | 11,40 |
| Plantacare® 818 UP, Cognis, 51%-ig, (INCI: Coco Glucoside) | 5,60 |
| Water | 63,00 |
| | |
| TEGOSOFT® LSE 65 K SOFT, Evonik Goldschmidt GmbH, (INCI: Sucrose Cocoate) | 1,50 |
| TEGO® Betain F 50, Evonik Goldschmidt GmbH, 38%-ig, (INCI: Cocamidopropyl Betaine) | 18,00 |
| Perfume,preservative | q.s. |
| Citric Acid, 30% | q.s. |
| N-(2-Hydroxypropyl)-12-Hydroxystearamid | 0,50 |

**Tabelle 4: Formulierungsbeispiel 3, feuchtigkeitspendendes Hautreinigungsmittel**

| | | |
|---|---|---|
| A | TEXAPON® NSO, Cognis, 28%-ig, (INCI: Sodium Laureth Sulfate) | 30,00 |
| | Perfume | 0,30 |
| B | Water | 54,80 |
| | TEGOCEL® fluid HPM 4000, Evonik Goldschmidt GmbH, (INCI: Hydroxypropyl Methylcellulose) | 1,20 |
| | TEGO® Betain C 60, Evonik Goldschmidt GmbH, 46%-ig, (INCI: Cocamidopropyl Betaine) | 8,10 |
| | TEGOSOFT® APM, Evonik Goldschmidt GmbH, (INCI: PPG-3 Myristyl Ether) | 1,00 |
| | TEGO® Pearl N 300, Evonik Goldschmidt GmbH, (INCI: Glycol Distearate; Laureth-4; Cocamidopropyl Betaine) | 2,00 |
| | REWODERM® LI S 80, Evonik Goldschmidt GmbH, (INCI: PEG-200 Hydrogenated Glyceryl Palmate; PEG-7 Glyceryl Cocoate) | 1,00 |
| | Preservative | 0,60 |
| | Citric Acid, 30%-ig | q.s. |
| N-(2-Hydroxypropyl)-12-Hydroxystearamid | | 1,00 |

**Tabelle 5: Formulierungsbeispiel 4, Duschgel**

| | |
|---|---|
| TAGAT® CH 40, Evonik Goldschmidt GmbH, (INCI: PEG-40 Hydrogenated Castor Oil) | 2,50 |
| Perfume | 0,30 |
| TEXAPON® NSO, Cognis, 28%-ig, (INCI: Sodium Laureth Sulfate) | 42,90 |
| Water | 39,30 |
| TEGO® Betain F 50, Evonik Goldschmidt GmbH, 38%-ig, (INCI: Cocamidopropyl Betaine) | 10,70 |
| LACTIL®, Evonik Goldschmidt GmbH, (INCI: Sodium Lactate; Sodium PCA; Glycine; Fructose; Urea; Niacinamide; Inositol; Sodiumbenzoate; Lactic Acid) | 1,00 |
| ANTIL® 171, Evonik Goldschmidt GmbH, (INCI: PEG-18 Glyceryl Oleate/Cocoate) | 2,00 |
| Preservative | 0,30 |
| N-(2-aminoethyl)-12-Hydroxystearamid | 1,00 |

**Tabelle 6: Formulierungsbeispiel 5, Duschgel**

| | |
|---|---|
| TEXAPON® NSO, Cognis, 28%-ig, (INCI: Sodium Laureth Sulfate) | 37,00 |
| Perfume | 0,30 |
| Water | 42,00 |
| REWOTERIC® AMC, Evonik Goldschmidt GmbH, 32%-ig, (INCI: Sodium Cocoamphoacetate) | 9,00 |
| TEGO® Betain 810, Evonik Goldschmidt GmbH, 38%-ig, (INCI: Capryl/Capramidopropyl Betaine) | 7,60 |
| LACTIL®, Evonik Goldschmidt GmbH, (INCI: Sodium Lactate; Sodium PCA; Glycine; Fructose; Urea; Niacinamide; Inositol; Sodiumbenzoate; Lactic Acid) | 1,00 |
| Citric Acid, 30%-ig | 1,30 |
| REWODERM® LI S 80, Evonik Goldschmidt GmbH, (INCI: PEG-200 Hydrogenated Glyceryl Palmate; PEG-7 Glyceryl Cocoate) | 0,50 |
| Preservative | 0,30 |
| N-(2-aminoethyl)-12-Hydroxystearamid | 1,00 |

**Tabelle 7: Formulierungsbeispiel 6, Shampoo, PEG- & sulfate free**

| | |
|---|---|
| REWOTERIC® AM C, Evonik Goldschmidt GmbH, 32%-ig, (INCI: Sodium Cocoamphoacetate) | 15,00 |
| Plantapon ACG 50, Cognis (INCI: Disodium Cocoyl Glutamate) | 3,80 |
| Perfume | 0,30 |
| Water | 66,30 |
| TEGO® Betain F 50, Evonik Goldschmidt GmbH, 38%-ig, (INCI: Cocamidopropyl Betaine) | 10,00 |
| VARISOFT® PATC, Evonik Goldschmidt GmbH, (INCI: Palmitamidopropyltrimonium Chloride) | 2,30 |
| REWOMID® SPA, Evonik Goldschmidt GmbH, (INCI: Isostearamide MIPA) | 1,00 |
| Preservative | 0,30 |
| Citric Acid, 30 %-ig | q.s. |
| N-(2-Hydroxypropyl)-12-Hydroxystearamid | 1,00 |

**Tabelle 8: Formulierungsbeispiel 7, Duschgel**

| | |
|---|---|
| TEXAPON® NSO, Cognis, 28%-ig (INCI: Sodium Laureth Sulfate) | 15,00 |
| Perfume | 0,30 |
| PGFAC-S, Cognis (INCI: Sodium cocoyl hydrolyzed wheat protein glutamate) | 1,50 |
| REWOPOL SB CS 50 B, Evonik Goldschmidt GmbH, 40%-ig, (INCI: Disodium PEG-5 Laurylcitrate Sulfosuccinate; Sodium Laureth Sulfate) | 7,50 |
| Water | 59,10 |
| TEGO® Betain F 50, Evonik Goldschmidt GmbH, 38%-ig, (INCI: Cocamidopropyl Betaine) | 9,00 |
| TEGO® Betain 810, Evonik Goldschmidt GmbH, 38%-ig, (INCI: Capryl/Capramidopropyl Betaine) | 4,00 |
| ANTIL® 200, Evonik Goldschmidt GmbH, (INCI: PEG-200 Hydrogenated Glyceryl Palmate; PEG-7 Glyceryl Cocoate) | 1,80 |
| Preservative | 0,30 |
| N-(N,N-Dimethyl-3-aminopropyl)-12-Hydroxystearamid | 1,50 |

**Tabelle 9: Formulierungsbeispiel 8, Shampoo, PEG- & sulfate free**

| | | |
|---|---|---|
| A | REWOTERIC® AMC, Evonik Goldschmidt GmbH, 32%-ig, (INCI: Sodium Cocoamphoacetate) | 20,00 |
| | REWOPOL® SB F 12 P, Evonik Goldschmidt, 96%-ig, (INCI: Disodium Lauryl Sulfosuccinate) | 5,90 |
| B | Water | 65,90 |
| | Citric Acid, 30%-ig | 3,60 |
| C | ANTIL® HS 60, Evonik Goldschmidt GmbH, (INCI: Cocamidopropyl Betaine; Glyceryl Laurate) | 3,00 |
| | Preservative | 0,60 |
| N-(N,N-Dimethyl-3-aminopropyl)-12-Hydroxystearamid | | 1,00 |

**Tabelle 10: Formulierungsbeispiel 9, Körperreinigungsmittel**

| | | |
|---|---|---|
| A | TEXAPON® NSO Cognis 28%-ig,(INCI: Sodium Laureth Sulfate | 30,00 |
| | | |
| | ABIL® B 8832, Evonik Goldschmidt GmbH, (INCI: Bis-PEG/PPG-20/20 Dimethicone) | 0,30 |
| | Perfume | 0,30 |
| B | Water | 52,50 |
| | TEGOCEL® fluid HPM 4000, Evonik Goldschmidt GmbH, (INCI: Hydroxypropyl Methylcellulose) | 1,20 |
| | Citric Acid Monohydrate | 0,50 |
| | REWOTERIC® AM C, Evonik Goldschmidt GmbH, 32%-ig, (INCI: Sodium Cocoamphoacetate) | 10,00 |
| | TEGO® Pearl N 300, Evonik Goldschmidt GmbH, (INCI: Glycol Distearate; Laureth-4; Cocamidopropyl Betaine) | 2,00 |
| | REWODERM® LI S 80, Evonik Goldschmidt GmbH, (INCI: PEG-200 Hydrogenated Glyceryl Palmate; PEG-7 Glyceryl Cocoate) | 1,60 |
| | Preservative | 0,60 |
| | Citric Acid, 30%-ig | q.s. |
| N-(2-Hydroxypropyl)-12-Hydroxystearamid | | 1,00 |

**Tabelle 11: Formulierungsbeispiel 10, Sprühbare Haarmilch, PEG-free**

| | | |
|---|---|---|
| A | Water | 95,30 |
| | Lactic Acid, 80%-ig | 0,40 |
| B | TEGO® AMID S 18, Evonik Goldschmidt GmbH, (INCI: Stearamidopropyl Dimethylamine) | 1,20 |
| | TEGIN® G 1100 Pellets, Evonik Goldschmidt GmbH, (INCI: Glycol Distearate) | 0,60 |
| | TEGO® Care PS, Evonik Goldschmidt GmbH, (INCI: Methyl Glucose Sesquistearate) | 1,20 |
| | TEGOSOFT® DEC, Evonik Goldschmidt GmbH, (INCI: Diethylhexyl Carbonate) | 0,30 |
| | Perfume, preservative | q.s. |
| N-(2-aminoethyl)-12-Hydroxystearamid | | 1,00 |

**Tabelle 12: Formulierungsbeispiel 11, Body cleansing Foam**

| | |
|---|---|
| TEXAPON® NSO, Cognis, 28%-ig (INCI: Sodium Laureth Sulfate) | 14 |
| Perfume | 0,3 |
| REWOTERIC® AM C, Evonik Goldschmidt GmbH, 32%-ig (INCI: Sodium Cocoamphoacetate) | 8 |
| Water | 75,0 |
| TEGOCEL® HPM 50, Evonik Goldschmidt GmbH (INCI: Hydroxypropyl Methylcellulose) | 0,5 |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 1 |
| Citric Acid Monohydrate | 0,5 |
| N-(2-Hydroxypropyl)-12-Hydroxystearamid | 0,7 |

**Tabelle 13: Formulierungsbeispiel 12, Conditioning Shampoo**

| | |
|---|---|
| TEXAPON® NSO, Cognis, 28%-ig (INCI: Sodium Laureth Sulfate) | 32,00 |
| VARISOFT® PATC, Evonik Goldschmidt GmbH(INCI: Palmitamidopropyltrimonium Chloride) | 1,50 |
| REWODERM® LI S 80, Evonik Goldschmidt GmbH (INCI: PEG-200 Hydrogenated Glyceryl Palmate; PEG-7 Glyceryl Cocoate) | 2,00 |
| Perfume | 0,25 |
| Water | 53,75 |
| TEGO® Cosmo C 100, Evonik Goldschmidt GmbH, (INCI: Creatine) | 1,00 |
| Jaguar C-162, Rhodia (INCI: Hydroxypropyl Guar Hydroxypropyltrimonium Chloride) | 0,20 |
| TEGO® Betain F 50, Evonik Goldschmidt GmbH, 38%-ig (INCI: Cocamidopropyl Betaine) | 8,00 |
| NaCl | 0,50 |
| Preservative | q.s. |
| N-(2-Hydroxypropyl)-12-Hydroxystearamid | 0,80 |

**Tabelle 14: Formulierungsbeispiel 13, Pearlized Shampoo**

| | |
|---|---|
| TEXAPON® NSO, Cognis, 28%-ig (INCI: Sodium Laureth Sulfate) | 32,00 |
| Perfume | 0,25 |
| Water | 54,75 |
| TEGO® Betain F 50, Evonik Goldschmidt GmbH, 38%-ig (INCI: Cocamidopropyl Betaine) | 8,00 |
| TEGO® Pearl N 300 Evonik Goldschmidt GmbH (INCI: Glycol Distearate; Laureth-4; Cocamidopropyl Betaine) | 2,00 |
| ANTIL® 171 Evonik Goldschmidt GmbH (INCI: PEG-18 Glyceryl Oleate/Cocoate) | 1,50 |
| NaCl | 0,50 |
| Preservative | q.s. |
| N-(N,N-Dimethyl-3-aminopropyl)-12-Hydroxystearamid | 1,00 |

**Tabelle 15: Formulierungsbeispiel 14, Rinse-Off Conditioner**

| | |
|---|---|
| Water | 90,00 |
| VARISOFT® EQ 65, Evonik Goldschmidt GmbH (INCI: Distearyl Dimonium Chloride, Cetearyl Alcohol) | 2,00 |
| VARISOFT® BT 85, Evonik Goldschmidt GmbH (INCI: Behentrimonium Chloride) | 2,00 |
| TEGO® Alkanol 1618, Evonik Goldschmidt GmbH (INCI: Cetearyl Alcohol) | 5,00 |
| Preservative, Perfume | q.s. |
| N-(N,N-Dimethyl-3-aminopropyl)-12-Hydroxystearamid | 1,00 |

**Tabelle 16: Formulierungsbeispiel 15, Conditioning Shampoo**

| | |
|---|---|
| TEXAPON® NSO, Cognis, 28%-ig (INCI: Sodium Laureth Sulfate) | 32,00 |
| ANTIL® 200, Evonik Goldschmidt GmbH (INCI: PEG-200 Hydrogenated Glyceryl Palmate; PEG-7 Glyceryl Cocoate) | 2,00 |
| Perfume | 0,25 |
| Water | 56,25 |
| Polymer JR 400, Amerchol (INCI: Polyquaternium-10) | 0,20 |
| TEGO® Betain F 50, Evonik Goldschmidt GmbH, 38%-ig (INCI: Cocamidopropyl Betaine) | 8,00 |
| NaCl | 0,30 |
| Preservative | q.s. |
| N-(2-Hydroxypropyl)-12-Hydroxystearamid | 1,00 |

**Tabelle 17: Formulierungsbeispiel 16, feuchtigkeitspendendes Hautreinigungsmittel**

| | | |
|---|---|---|
| A | TEXAPON® NSO, Cognis, 28%-ig, (INCI: Sodium Laureth Sulfate) | 30,00 |
| | Cap01 | 0,70 |
| | Perfume | 0,30 |
| B | Water | 55,10 |
| | TEGOCEL® fluid HPM 4000, Evonik Goldschmidt GmbH, (INCI: Hydroxypropyl Methylcellulose) | 1,20 |
| | TEGO® Betain C 60, Evonik Goldschmidt GmbH, 46%-ig, (INCI: Cocamidopropyl Betaine) | 8,10 |
| | TEGOSOFT® APM, Evonik Goldschmidt GmbH, (INCI: PPG-3 Myristyl Ether) | 1,00 |
| | Cutina TS, Cognis (INCI:PEG- 3 Distearate) | 1,00 |
| | REWODERM® LI S 80, Evonik Goldschmidt GmbH, (INCI: PEG-200 Hydrogenated Glyceryl Palmate; PEG-7 Glyceryl Cocoate) | 1,00 |
| | Preservative | 0,60 |
| | Citric Acid, 30%-ig | q.s. |
| N-(2-Hydroxypropyl)-12-Hydroxystearamid | | 1,00 |

**Tabelle 18: Formulierungsbeispiel 17, Duschgel**

| | |
|---|---|
| TEXAPON® NSO, Cognis, 28%-ig (INCI: Sodium Laureth Sulfate) | 15,00 |
| Perfume | 0,30 |
| PGFAC-S, Cognis (INCI: Sodium cocoyl hydrolyzed wheat protein glutamate) | 1,50 |
| REWOPOL SB CS 50 B, Evonik Goldschmidt GmbH, 40%-ig, (INCI: Disodium PEG-5 Laurylcitrate Sulfosuccinate; Sodium Laureth Sulfate) | 7,50 |
| Water | 59,35 |
| TEGO® Betain F 50, Evonik Goldschmidt GmbH, 38%-ig, (INCI: Cocamidopropyl Betaine) | 9,00 |
| TEGO® Betain 810, Evonik Goldschmidt GmbH, 38%-ig, (INCI: Capryl/Capramidopropyl Betaine) | 4,00 |
| Polyquaternium-7, Nalco, (INCI: Merquat 550) | 0,50 |
| ANTIL® 200, Evonik Goldschmidt GmbH, (INCI: PEG-200 Hydrogenated Glyceryl Palmate; PEG-7 Glyceryl Cocoate) | 1,80 |
| Preservative | 0,30 |
| N-(2-Hydroxypropyl)-12-Hydroxystearamid | 0,75 |

**Tabelle 19: Formulierungsbeispiel 18, Körperreinigungsmittel**

| | | |
|---|---|---|
| A | TEXAPON® NSO Cognis 28%-ig,(INCI: Sodium Laureth Sulfate | 30,00 |
| | ABIL® B 8832, Evonik Goldschmidt GmbH, (INCI: Bis-PEG/PPG-20/20 Dimethicone) | 0,30 |
| | Perfume | 0,30 |
| B | Water | 52,50 |
| | TEGOCEL® fluid HPM 4000, Evonik Goldschmidt GmbH, (INCI: Hydroxypropyl Methylcellulose ) | 1,20 |
| | Citric Acid Monohydrate | 0,50 |
| | REWOTERIC® AM C, Evonik Goldschmidt GmbH, 32%-ig, (INCI: Sodium Cocoamphoacetate) | 10,00 |
| | Cutina TS, Cognis (INCI:PEG-3 Distearate) | 2,00 |
| | REWODERM® LI S 80, Evonik Goldschmidt GmbH, (INCI: PEG-200 Hydrogenated Glyceryl Palmate; PEG-7 Glyceryl Cocoate) | 1,60 |
| | Preservative | 0,60 |
| | Citric Acid, 30%-ig | q.s. |
| N-(2-aminoethyl)-12-Hydroxystearamid | | 1,00 |

**Tabelle 20: Formulierungsbeispiel 19, Body cleansing Foam**

| | |
|---|---|
| TEXAPON® NSO, Cognis, 28%-ig (INCI: Sodium Laureth Sulfate) | 14 |
| Perfume | 0,3 |
| REWOTERIC® AM C, Evonik Goldschmidt GmbH, 32%-ig (INCI: Sodium Cocoamphoacetate) | 8 |
| Water | 74,7 |
| TEGOCEL® HPM 50, Evonik Goldschmidt GmbH (INCI: Hydroxypropyl Methylcellulose) | 0,5 |
| LACTIL®, Evonik Goldschmidt GmbH (INCI: Sodium Lactate; Sodium PCA; Glycine; Fructose; Urea; Niacinamide; Inositol; Sodium benzoate; Lactic Acid) | 1 |
| Panthenol, BASF, (INCI: D-Panthenol USP) | 0,2 |
| Citric Acid Monohydrate | 0,5 |
| N-(2-Hydroxypropyl)-12-Hydroxystearamid | 0,8 |

**Tabelle 21: Formulierungsbeispiel 20, Rinse-Off Conditioner**

| | |
|---|---|
| Water | 89,40 |
| VARISOFT® EQ 65, Evonik Goldschmidt GmbH (INCI: Distearyl Dimonium Chloride, Cetearyl Alcohol) | 2,00 |
| VARISOFT® BT 85, Evonik Goldschmidt GmbH (INCI: Behentrimonium Chloride) | 2,00 |
| | |
| TEGO® Alkanol 16, Evonik Goldschmidt GmbH (INCI:Cetyl Alkohol) | 5,00 |
| Preservative, Perfume | q.s. |
| N-(N,N-Dimethyl-3-aminopropyl)-12-Hydroxystearamid | 1,6 |

**Tabelle 22: Formulierungsbeispiel 21, Rinse-Off Conditioner**

| | |
|---|---|
| Water | 89,40 |
| VARISOFT® EQ 65, Evonik Goldschmidt GmbH (INCI: Distearyl Dimonium Chloride, Cetearyl Alcohol) | 2,00 |
| VARISOFT® BT 85, Evonik Goldschmidt GmbH (INCI: Behentrimonium Chloride) | 2,00 |
| TEGO® Alkanol 18, Evonik Goldschmidt GmbH, (INCI: Stearyl Alkohol) | 5,00 |
| Preservative, Perfume | q.s. |
| N-(N,N-Dimethyl-3-aminopropyl)-12-Hydroxystearamid | 1,6 |

**Tabelle 23: Formulierungsbeispiel 22, Rinse-Off Conditioner**

| | |
|---|---|
| Water | 88,40 |
| VARISOFT® EQ 65, Evonik Goldschmidt GmbH (INCI: Distearyl Dimonium Chloride, Cetearyl Alcohol) | 2,00 |
| VARISOFT® BT 85, Evonik Goldschmidt GmbH (INCI: Behentrimonium Chloride) | 2,00 |
| TEGO® Alkanol 1618, Evonik Goldschmidt GmbH (INCI: Cetearyl Alcohol) | 5,00 |
| DC 949, Dow Corning, (INCI: Amodimethicone) | 1,00 |
| Preservative, Perfume | q.s. |
| N-(N,N-Dimethyl-3-aminopropyl)-12-Hydroxystearamid | 1,6 |

**Tabelle 24: Formulierungsbeispiel 23, Rinse-Off Conditioner**

| | |
|---|---|
| Water | 88,40 |
| VARISOFT® EQ 65, Evonik Goldschmidt GmbH (INCI: Distearyl Dimonium Chloride, Cetearyl Alcohol) | 2,00 |
| VARISOFT® BT 85, Evonik Goldschmidt GmbH (INCI: Behentrimonium Chloride) | 2,00 |
| TEGO® Alkanol 1618, Evonik Goldschmidt GmbH (INCI: Cetearyl Alcohol) | 5,00 |
| DC 1503 Fluid, Dow Corning, (INCI: Dimethicone, Dimethiconol) | 1,00 |
| Preservative, Perfume | q.s. |
| N-(N,N-Dimethyl-3-aminopropyl)-12-Hydroxystearamid | 1,6 |

**Tabelle 25: Formulierungsbeispiel 24, Turbid Conditioning Shampoo**

| | |
|---|---|
| TEXAPON® NSO, Cognis, 28%-ig (INCI: Sodium Laureth Sulfate) | 32,00 |
| ANTIL® 200, Evonik Goldschmidt GmbH (INCI: PEG-200 Hydrogenated Glyceryl Palmate; PEG-7 Glyceryl Cocoate) | 2,00 |
| Perfume | 0,25 |
| Water | 53,25 |
| Polymer JR 400, Amerchol (INCI: Polyquaternium-10) | 0,20 |
| TEGO® Betain F 50, Evonik Goldschmidt GmbH, 38%-ig (INCI: Cocamidopropyl Betaine) | 8,00 |
| DC1503 Fluid, Dow Corning, (INCI: Dimethicone, Dimethiconol) | 1,00 |
| TEGO® Pearl N 300 Evonik Goldschmidt GmbH (INCI: Glycol Distearate; Laureth-4; Cocamidopropyl Betaine) | 2,00 |
| NaCl | 0,30 |
| Preservative | q.s. |
| N-(2-Hydroxypropyl)-12-Hydroxystearamid | 1,00 |

**Tabelle 26: Formulierungsbeispiel 25, Conditioning Anti- Dandruff Sham**

| | | |
|---|---|---|
| A | TEGIN® G 1100 Pellets, Evonik Goldschmidt GmbH, (INCI: Glycol Distearate) | 3,00 |
| | TEXAPON® NSO, Cognis, 28%-ig (INCI: Sodium Laureth Sulfate) | 40,0 |
| B | Perfume | 0,30 |
| | Zinc-Pyrion NF, WeylChem, 48%ig (INCI: Zinc Pyrithione) | 2,00 |
| | ABIL® Quat 3272, Evonik Goldschmidt GmbH, (INCI: Quaternium- 80) | 1,00 |
| C | Water | 37,4 |
| | TEGO® Carbomer 341 ER, Evonik Goldschmidt GmbH, (INCI: Acrylates/ C10-30 Alkyl Acrylate Crosspolymer) | 0,20 |
| | Polymer JR 400, Amerchol, (INCI: Polyquaternium-10) | 0,30 |
| | NaOH, 25%ig | 0,30 |
| D | Rewoteric AM B U 185® Evonik Goldschmidt GmbH, (INCI: Undecylenamidopropyl Betaine) | 12,5 |
| | Preservative | q.s |
| N-(2-Hydroxypropyl)-12-Hydroxystearamid | | 3,00 |

**Tabelle 27: Formulierungsbeispiel 26, Conditioning Anti- Dandruff Sham**

| | | |
|---|---|---|
| A | TEGIN® G 1100 Pellets, Evonik Goldschmidt GmbH, (INCI: Glycol Distearate) | 3,00 |
| | TEXAPON® NSO, Cognis, 28%-ig (INCI: Sodium Laureth Sulfate) | 40,0 |
| B | Perfume | 0,30 |
| | Crinipan AD, Haarmann & Reimer Fragrance GmbH (INCI: Climbazol) | 0,30 |
| | ABIL® Quat 3272, Evonik Goldschmidt GmbH, (INCI: Quaternium- 80) | 1,00 |
| C | Water | 39,1 |
| | TEGO® Carbomer 341 ER, Evonik Goldschmidt GmbH, (INCI: Acrylates/ C10-30 Alkyl Acrylate Crosspolymer) | 0,20 |
| | Polymer JR 400, Amerchol, (INCI: Polyquaternium-10) | 0,30 |
| | NaOH, 25%ig | 0,30 |
| D | Rewoteric AM B U 185® Evonik Goldschmidt GmbH, (INCI: Undecylenamidopropyl Betaine) | 12,5 |
| | Preservative | q.s |
| N-(2-Hydroxypropyl)-12-Hydroxystearamid | | 3,00 |

**Tabelle 28: Formulierungsbeispiel 27, Conditioning Anti- Dandruff Sham**

| | | |
|---|---|---|
| A | TEGIN® G 1100 Pellets, Evonik Goldschmidt GmbH, (INCI: Glycol Distearate) | 3,00 |
| | TEXAPON® NSO, Cognis, 28%-ig (INCI: Sodium Laureth Sulfate) | 40,0 |
| B | Perfume | 0,30 |
| | Zinc-Pyrion NF, WeylChem, 48%ig (INCI: Zinc Pyrithione) | 2,00 |
| | ABIL® Quat 3272, Evonik Goldschmidt GmbH, (INCI: Quaternium- 80) | 1,00 |
| C | Water | 37,1 |
| | TEGO® Carbomer 140, Evonik Goldschmidt GmbH, (INCI: Carbomer) | 0,50 |
| | Polymer JR 400, Amerchol, (INCI: Polyquaternium-10) | 0,30 |
| | NaOH, 25%ig | 0,30 |
| D | Rewoteric AM B U 185® Evonik Goldschmidt GmbH, (INCI: Undecylenamidopropyl Betaine) | 12,5 |
| | Preservative | q.s |
| N-(2-Hydroxypropyl)-12-Hydroxystearamid | | 3,00 |

**Tabelle 29: Formulierungsbeispiel 28, Conditioning Anti- Dandruff Sham**

| | | |
|---|---|---|
| A | TEGIN® G 1100 Pellets, Evonik Goldschmidt GmbH, (INCI: Glycol Distearate) | 3,00 |
| | TEXAPON® NSO, Cognis, 28%-ig (INCI: Sodium Laureth Sulfate) | 40,0 |
| B | Perfume | 0,30 |
| | Piroctone Olamine, Clariant (INCI: Octoprirox) | 0,30 |
| | ABIL® Quat 3272, Evonik Goldschmidt GmbH, (INCI: Quaternium- 80) | 1,00 |
| C | Water | 39,1 |
| | TEGO® Carbomer 341 ER, Evonik Goldschmidt GmbH, (INCI: Acrylates/ C10-30 Alkyl Acrylate Crosspolymer) | 0,20 |
| | Polymer JR 400, Amerchol, (INCI: Polyquaternium-10) | 0,30 |
| | NaOH, 25%ig | 0,30 |
| D | Rewoteric AM B U 185® Evonik Goldschmidt GmbH, (INCI: Undecylenamidopropyl Betaine) | 12,5 |
| | Preservative | q.s |
| N-(2-Hydroxypropyl)-12-Hydroxystearamid | | 3,00 |

## Patentansprüche

1. Verbindung der allgemeinen Formel (I) 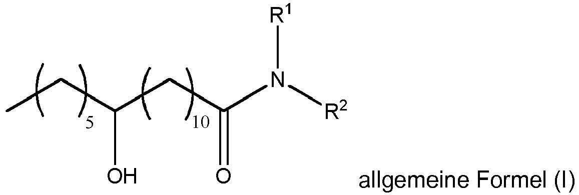 mit
R¹ = N-(2-Hydroxyethyl)-aminoethyl- und R² = N-(2-Hydroxyethyl)-aminoethyl-,
R¹ = 2-(2-Hydroxyethoxy)ethyl- und R² = 2-(2-Hydroxyethoxy)ethyl-,
R¹ = Methyl- oder 2,3,4,5,6-pentahydroxyhexyl- und R² = 2,3,4,5,6-pentahydroxyhexyl-, wobei R¹ bevorzugt = Methyl- und
R¹ = H- oder 2-(1-Piperazinyl)ethyl- und R² 2-(1-Piperazinyl)ethyl-, wobei R¹ bevorzugt = H.

2. Wässrige Formulierung mit einem Wassergehalt von mindestens 20 Gew.-% bezogen auf die Gesamtformulierung enthaltend mindestens eine Verbindung der allgemeinen Formel (I) 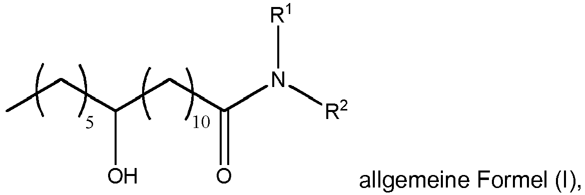 **dadurch gekennzeichnet, dass** die Verbindung der allgemeinen Formel (I) ausgewählt ist aus solchen mit
R¹ = H oder 2-Hydroxypropyl - und R² = 2-Hydroxypropyl-, wobei R¹ bevorzugt = H,
R¹ = H oder 2-Aminoethyl- und R² = 2-Aminoethyl-, wobei R¹ bevorzugt = H,
R¹ = H oder N-(2-Hydroxyethyl)-aminoethyl- und R² = N-(2-Hydroxyethyl)-aminoethyl-, wobei R¹ bevorzugt = H,
R¹ = H oder 2-(2-Hydroxyethoxy)ethyl- und R² = 2-(2-Hydroxyethoxy)ethyl-, wobei R¹ bevorzugt = H,
R¹ = Methyl- oder 2,3,4,5,6-pentahydroxyhexyl- und R² = 2,3,4,5,6-pentahydroxyhexyl-, wobei R¹ bevorzugt = Methyl- und
R¹ = H- oder 2-(1-Piperazinyl)ethyl- und R² = 2-(1-Piperazinyl)ethyl-, wobei R¹ bevorzugt = H.

3. Wässrige Formulierung gemäß Anspruch 2, **dadurch gekennzeichnet, dass** sie mindestens ein Tensid enthält, bevorzugt in einer Menge von 5 Gew.-% bis 60 Gew.-%, besonders bevorzugt von 15 Gew.-% bis 40 Gew.-%, wobei sich die Gew.-% auf die Gesamtformulierung beziehen.

4. Wässrige Formulierung gemäß Anspruch 3, **dadurch gekennzeichnet, dass** das mindestens eine Tensid eine Mischung aus mindestens einem anionischen und mindestens einem nichtionischen Tensid ist.

5. Wässrige Formulierung gemäß Anspruch 3, **dadurch gekennzeichnet, dass** das mindestens eine Tensid eine Biotensid ausgewählt aus Rhamnolipiden, Sophorolipiden, Trehalose- und andere mycolsäurehaltige Glycolipiden sowie Cellobiose- und Mannosylerythritollipiden ist.

6. Wässrige Formulierung gemäß mindestens einem der Ansprüche 2 bis 5, **dadurch gekennzeichnet, dass** sie mindestens ein Enzym enthält, insbesondere ausgewählt aus der Klasse der Hydrolasen.

7. Wässrige Formulierung gemäß mindestens einem der Ansprüche 2 bis 6, **dadurch gekennzeichnet, dass** die mindestens eine Verbindung der allgemeinen Formel (I) in einer Menge von 0,1 Gew.-% bis 50 Gew.-%, besonders bevorzugt von 0,5 Gew.-% bis 25 Gew.-%, wobei sich die Gew.-% auf die Gesamtformulierung beziehen, enthält.

8. Verfahren zur Herstellung von verdickten, wässrigen Formulierungen umfassend die Verfahrensschritte:
A) Bereitstellen einer Verbindung der allgemeinen Formel (I) 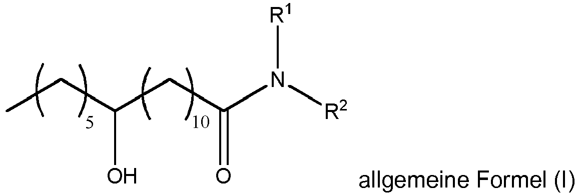
mit R¹ ausgewählt aus H oder einem gegebenenfalls OH- oder Aminogruppen substituiertem und/oder mit Sauerstoff oder Aminogruppen unterbrochenem Alkylrest mit 1 bis 6 Kohlenstoffatomen und
mit R² ausgewählt aus H oder einem gegebenenfalls OH- oder Aminogruppen substituiertem und/oder mit Sauerstoff oder Aminogruppen unterbrochenem Alkylrest mit 1 bis 6 Kohlenstoffatomen,
B) Bereitstellen einer wässrigen Formulierung und
C) Mischen der Verbindung der allgemeinen Formel (I) mit der wässrigen Formulierung,
**dadurch gekennzeichnet, dass** in
Verfahrensschritt A) eine Verbindung der allgemeinen Formel (I) in Form einer Zusammensetzung aufweisend einen pH-Wert von 0 bis 4, insbesondere von 0,5 bis 2, besonders bevorzugt von 0,8 bis 1,5, eingesetzt wird, wobei mindestens einer der Reste R¹ oder R² ein mit mindestens einer Aminogruppen substituierter Alkylrest mit 1 bis 6 Kohlenstoffatomen ist, und in einem
Verfahrensschritt D) der pH-Wert der Mischung aus C) auf einen pH-Bereich von 5 bis 12, besonders bevorzugt auf einen pH-Bereich von 7 bis 10, erhöht wird.

9. Verfahren gemäß Anspruch 8, **dadurch gekennzeichnet, dass** in Verfahrensschritt B) das Mischen in einem Temperaturbereich von 65 °C bis 100 °C, bevorzugt von 75 °C bis 85 °C durchgeführt wird, und in einem Verfahrensschritt E) die Mischung aus C) auf einen Temperatur-Bereich von 0 °C bis 60 °C, besonders bevorzugt auf einen Temperatur-Bereich von 5 °C bis 45 °C, abgesenkt wird.

10. Verwendung einer Verbindung der allgemeinen Formel (I) 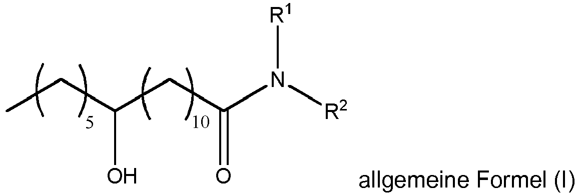
mit R¹ ausgewählt aus H oder einem gegebenenfalls OH- oder Aminogruppen substituiertem und/oder mit Sauerstoff oder Aminogruppen unterbrochenem Alkylrest mit einem bis 6 Kohlenstoffatomen und
mit R² ausgewählt aus H oder einem gegebenenfalls OH- oder Aminogruppen substituiertem und/oder mit Sauerstoff oder Aminogruppen unterbrochenem Alkylrest mit einem bis 6 Kohlenstoffatomen,
zur Verdickung von wässrigen Formulierungen,
**dadurch gekennzeichnet, dass** die Verbindung der allgemeinen Formel (I) ausgewählt ist aus solchen mit
R¹ = H oder 2-Hydroxypropyl - und R² = 2-Hydroxypropyl-, wobei R¹ bevorzugt = H,
R¹ = H oder 2-Hydroxyethyl- und R² = 2-Hydroxyethyl-, wobei R¹ bevorzugt = H,
R¹ = H oder 2-Aminoethyl- und R² = 2-Aminoethyl-, wobei R¹ bevorzugt = H,
R¹ = H oder N-(2-Hydroxyethyl)-aminoethyl- und R² = N-(2-Hydroxyethyl)-aminoethyl-, wobei R¹ bevorzugt = H,
R¹ = H oder 2-(2-Hydroxyethoxy)ethyl- und R² = 2-2-(2-Hydroxyethoxy)ethyl-, wobei R¹ bevorzugt = H,
R¹ = Methyl- oder 2,3,4,5,6-pentahydroxyhexyl- und R² = 2,3,4,5,6-pentahydroxyhexyl-, wobei R¹ bevorzugt = Methyl-,
R¹ = H- oder 2-(1-Piperazinyl)ethyl- und R² = 2-(1-Piperazinyl)ethyl-, wobei R¹ bevorzugt = H und
R¹ = H oder N,N-Dimethyl-3-aminopropyl und R² = N,N-Dimethyl-3-aminopropyl, wobei R¹ bevorzugt = H.

## Claims

1. Compound of the general formula (I) 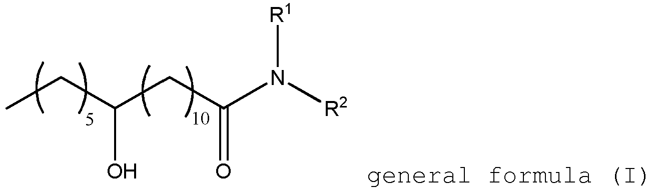 where
R¹ = N-(2-hydroxyethyl)aminoethyl and R² = N-(2-hydroxyethyl)aminoethyl,
R¹ = 2-(2-hydroxyethoxy)ethyl and R² = 2-(2-hydroxyethoxy)ethyl,
R¹ = methyl or 2,3,4,5,6-pentahydroxyhexyl and R² = 2,3,4,5,6-pentahydroxyhexyl, where R¹ preferably = methyl and
R¹ = H or 2-(1-piperazinyl)ethyl and R² 2-(1-piperazinyl)ethyl, where R¹ preferably = H.

2. Aqueous formulation with a water content of at least 20% by weight based on the total formulation, comprising at least one compound of the general formula (I) 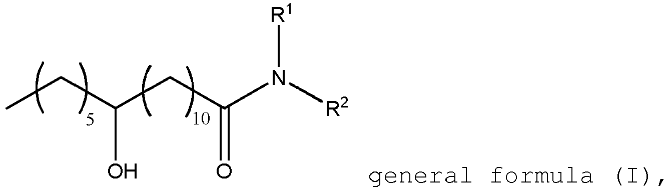 **characterized in that** the compound of the general formula (I) is selected from those where
R¹ = H or 2-hydroxypropyl and R² = 2-hydroxypropyl, where R¹ preferably = H,
R¹ = H or 2-aminoethyl and R² = 2-aminoethyl, where R¹ preferably = H,
R¹ = H or N-(2-hydroxyethyl)aminoethyl and R² = N-(2-hydroxyethyl)aminoethyl, where R¹ preferably = H,
R¹ = H or 2-(2-hydroxyethoxy)ethyl and R² = 2-(2-hydroxyethoxy)ethyl, where R¹ preferably = H,
R¹ = methyl or 2,3,4,5,6-pentahydroxyhexyl and R² = 2,3,4,5,6-pentahydroxyhexyl, where R¹ preferably = methyl and
R¹ = H or 2-(1-piperazinyl)ethyl and R² = 2-(1-piperazinyl)ethyl, where R¹ preferably = H.

3. Aqueous formulation according to Claim 2, **characterized in that** it comprises at least one surfactant, preferably in an amount of from 5% by weight to 60% by weight, particularly preferably from 15% by weight to 40% by weight, where the % by weight refer to the total formulation.

4. Aqueous formulation according to Claim 3, **characterized in that** the at least one surfactant is a mixture of at least one anionic and at least one nonionic surfactant.

5. Aqueous formulation according to Claim 3, **characterized in that** the at least one surfactant is a biosurfactant selected from rhamnolipids, sophorolipids, trehalose glycolipids and other mycolic acid-containing glucolipids and also cellobiose and mannosyl erythritol lipids.

6. Aqueous formulation according to at least one of Claims 2 to 5, **characterized in that** it comprises at least one enzyme, in particular selected from the class of hydrolases.

7. Aqueous formulation according to at least one of Claims 2 to 6, **characterized in that** it comprises at least one compound of the general formula (I) in an amount of from 0.1% by weight to 50% by weight, particularly preferably from 0.5% by weight to 25% by weight, where the % by weight refer to the total formulation.

8. Process for the preparation of thickened, aqueous formulations comprising the process steps:
A) provision of a compound of the general formula (I) 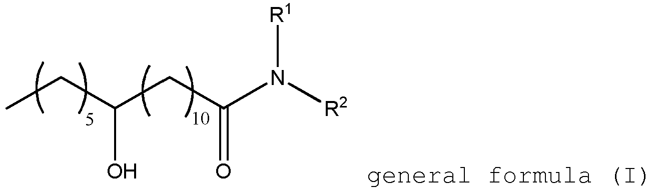
where R¹ is selected from H or an alkyl radical having 1 to 6 carbon atoms that is optionally substituted by OH or amino groups and/or interrupted with oxygen or amino groups and
where R² is selected from H or an alkyl radical having 1 to 6 carbon atoms that is optionally substituted by OH or amino groups and/or interrupted with oxygen or amino groups,
B) provision of an aqueous formulation and
C) mixing of the compound of the general formula (I) with the aqueous formulation
**characterized in that**, in
process step A), a compound of the general formula (I) is used in the form of a composition having a pH of from 0 to 4, in particular from 0.5 to 2, particularly preferably from 0.8 to 1.5, where at least one of the radicals R¹ or R² is an alkyl radical having 1 to 6 carbon atoms that is substituted with at least one amino group, and in a
process step D), the pH of the mixture from C) is increased to a pH range from 5 to 12, particularly preferably to a pH range from 7 to 10.

9. Process according to Claim 8, **characterized in that**, in
process step B), the mixing is carried out in a temperature range from 65°C to 100°C, preferably from 75°C to 85°C, and in a
process step E), the mixture from C) is reduced to a temperature range from 0°C to 60°C, particularly preferably to a temperature range from 5°C to 45°C.

10. Use of a compound of the general formula (I) 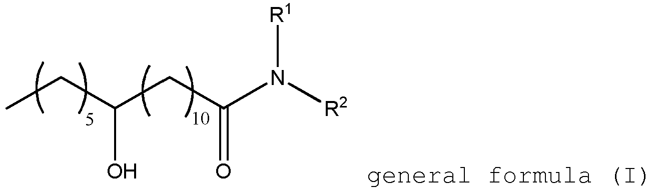
where R¹ is selected from H or an alkyl radical having 1 to 6 carbon atoms that is optionally substituted by OH or amino groups and/or interrupted with oxygen or amino groups and
where R² is selected from H or an alkyl radical having 1 to 6 carbon atoms that is optionally substituted by OH or amino groups and/or interrupted with oxygen or amino groups,
for thickening aqueous formulations,
**characterized in that** the compound of the general formula (I) is selected from those where
R¹ = H or 2-hydroxypropyl and R² = 2-hydroxypropyl, where R¹ preferably = H,
R¹ = H or 2-hydroxyethyl and R² = 2-hydroxyethyl, where R¹ preferably = H,
R¹ = H or 2-aminoethyl and R² = 2-aminoethyl, where R¹ preferably = H,
R¹ = H or N-(2-hydroxyethyl)aminoethyl and R² = N-(2-hydroxyethyl)aminoethyl, where R¹ preferably = H,
R¹ = H or 2-(2-hydroxyethoxy)ethyl and R² = 2-2-(2-hydroxyethoxy)ethyl, where R¹ preferably = H,
R¹ = methyl or 2,3,4,5,6-pentahydroxyhexyl and R² = 2,3,4,5,6-pentahydroxyhexyl, where R¹ preferably = methyl,
R¹ = H or 2-(1-piperazinyl)ethyl and R² = 2-(1-piperazinyl)ethyl, where R¹ preferably = H and
R¹ = H or N,N-dimethyl-3-aminopropyl and R² = N,N-dimethyl-3-aminopropyl, where R¹ preferably = H.

## Revendications

1. Composé de formule générale (I) 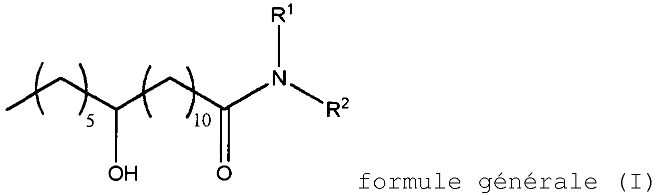 dans laquelle
R¹ = N-(2-hydroxyéthyl)-aminoéthyle et R² = N-(2-hydroxyéthyl)-aminoéthyle,
R¹ = 2-(2-hydroxyéthoxy)éthyle et R² = 2-(2-hydroxyéthoxy)éthyle,
R¹ = méthyle ou 2,3,4,5,6-pentahydroxyhexyle et R² = 2,3,4,5,6-pentahydroxyhexyle, R¹ représentant de préférence méthyle et
R¹ = H ou 2-(1-pipérazinyl)éthyle et R² 2-(1-pipérazinyl)éthyle, R¹ représentant de préférence H.

2. Formulation aqueuse présentant une teneur en eau d'au moins 20% en poids par rapport à la formulation totale, contenant au moins un composé de formule générale (I) 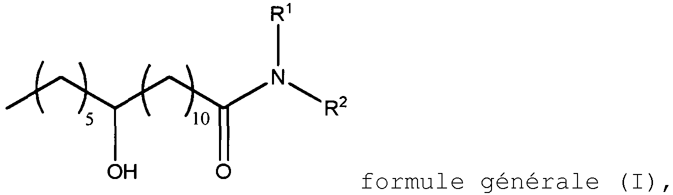 **caractérisé en ce que** le composé de formule générale (I) est choisi parmi ceux dans lesquels
R¹ = H ou 2-hydroxypropyle et R² = 2-hydroxypropyle, R¹ représentant de préférence H,
R¹ = H ou 2-aminoéthyle et R² = 2-aminoéthyle, R¹ représentant de préférence H,
R¹ = H ou N-(2-hydroxyéthyl)-aminoéthyle et R² = N-(2-hydroxyéthyl)-aminoéthyle, R¹ représentant de préférence H,
R¹ = H ou 2-(2-hydroxyéthoxy)éthyle et R² = 2-(2-hydroxyéthoxy)éthyle, R¹ représentant de préférence H,
R¹ = méthyle ou 2,3,4,5,6-pentahydroxyhexyle et R² = 2,3,4,5,6-pentahydroxyhexyle, R¹ représentant de préférence méthyle et
R¹ = H ou 2-(1-pipérazinyl)éthyle et R² = 2-(1-pipérazinyl)éthyle, R¹ représentant de préférence H.

3. Formulation aqueuse selon la revendication 2, **caractérisée en ce qu'**elle contient au moins un tensioactif, de préférence en une quantité de 5% en poids à 60% en poids, de manière particulièrement préférée de 15% en poids à 40% en poids, les % en poids se rapportant à la formulation totale.

4. Formulation aqueuse selon la revendication 3, **caractérisée en ce qu'**au moins un tensioactif est un mélange d'au moins un tensioactif anionique et d'au moins un tensioactif non ionique.

5. Formulation aqueuse selon la revendication 3, **caractérisée en ce qu'**au moins un tensioactif est un biotensioactif choisi parmi les rhamnolipides, les sophorolipides, les glycolipides contenant du tréhalose et autres acides mycoliques ainsi que les lipides de type cellobiose et de mannosylérythritol.

6. Formulation aqueuse selon au moins l'une quelconque des revendications 2 à 5, **caractérisée en ce qu'**elle contient au moins une enzyme, en particulier choisie dans la classe des hydrolases.

7. Formulation aqueuse selon au moins l'une quelconque des revendications 2 à 6, **caractérisée en ce qu'**elle contient au moins un composé de formule générale (I) en une quantité de 0,1% en poids à 50% en poids, de manière particulièrement préférée de 0,5% en poids à 25% en poids, les % en poids se rapportant à la formulation totale.

8. Procédé pour la préparation de formulations aqueuses épaissies, comprenant les étapes de procédé
A) préparation d'un composé de formule générale (I) 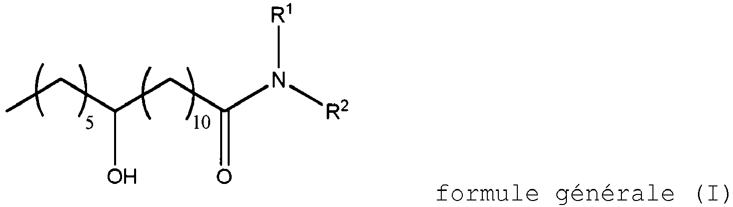
dans laquelle R¹ est choisi parmi H ou un radical alkyle, le cas échéant substitué par OH ou des groupes amino et/ou interrompu par oxygène ou des groupes amino, comprenant 1 à 6 atomes de carbone et
dans laquelle R² est choisi parmi H ou un radical alkyle, le cas échéant substitué par OH ou des groupes amino et/ou interrompu par oxygène ou des groupes amino, comprenant 1 à 6 atomes de carbone,
B) préparation d'une formulation aqueuse et
C) mélange du composé de formule générale (I) avec la formulation aqueuse, **caractérisé**
**en ce que** dans l'étape de procédé A), un composé de formule générale (I) est utilisé sous forme d'une composition présentant un pH de 0 à 4, en particulier de 0,5 à 2, de manière particulièrement préférée de 0,8 à 1,5, au moins un des radicaux R¹ ou R² étant un radical alkyle, substitué par au moins un groupe amino, comprenant 1 à 6 atomes de carbone, et, dans une étape de procédé D), le pH du mélange provenant de C) est augmenté à une plage de pH de 5 à 12, de manière particulièrement préférée à une plage de pH de 7 à 10.

9. Procédé selon la revendication 8, **caractérisé en ce que**, dans l'étape de procédé B), le mélange est réalisé dans une plage de température de 65°C à 100°C, de préférence de 75°C à 85°C et, dans une étape de procédé E), le mélange provenant de C) est abaissé à une plage de température de 0°C à 60°C, de manière particulièrement préférée à une plage de température de 5°C à 45°C.

10. Utilisation d'un composé de formule générale (I) 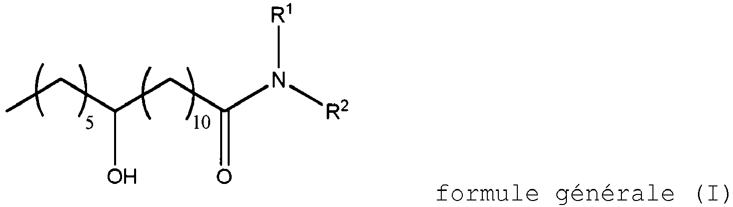
dans laquelle R¹ est choisi parmi H ou un radical alkyle, le cas échéant substitué par OH ou des groupes amino et/ou interrompu par oxygène ou des groupes amino, comprenant 1 à 6 atomes de carbone et
dans laquelle R² est choisi parmi H ou un radical alkyle, le cas échéant substitué par OH ou des groupes amino et/ou interrompu par oxygène ou des groupes amino, comprenant 1 à 6 atomes de carbone,
pour l'épaississement de formulations aqueuses, **caractérisé en ce que** le composé de formule générale (I) est choisi parmi ceux dans lesquels
R¹ = H ou 2-hydroxypropyle et R² = 2-hydroxypropyle, R¹ représentant de préférence H,
R¹ = H ou 2-hydroxyéthyle et R² = 2-hydroxyéthyle, R¹ représentant de préférence H,
R¹ = H ou 2-aminoéthyle et R² = 2-aminoéthyle, R¹ représentant de préférence H,
R¹ = H ou N-(2-hydroxyéthyl)-aminoéthyle et R² = N-(2-hydroxyéthyl)-aminoéthyle, R¹ représentant de préférence H,
R¹ = H ou 2-(2-hydroxyéthoxy)éthyle et R² = 2-2-(2-hydroxyéthoxy)éthyle, R¹ représentant de préférence H,
R¹ = méthyle ou 2,3,4,5,6-pentahydroxyhexyle et R² = 2,3,4,5,6-pentahydroxyhexyle, R¹ représentant de préférence méthyle,
R¹ = H ou 2-(1-pipérazinyl)éthyle et R² = 2-(1-pipérazinyl)éthyl-, R¹ représentant de préférence H et
R¹ = H ou N,N-diméthyl-3-aminopropyle et R² = N,N-diméthyl-3-aminopropyle, R¹ représentant de préférence H.
